(19) Europäisches Patentamt
European Patent Office
Office européen des brevets

(11) EP 2 618 220 A1

(12) EUROPEAN PATENT APPLICATION
published in accordance with Art. 153(4) EPC

(43) Date of publication:
**24.07.2013 Bulletin 2013/30**

(21) Application number: **11825171.9**

(22) Date of filing: **13.09.2011**

(51) Int Cl.:
*G03G 9/097* (2006.01) *C07C 321/28* (2006.01)
*G03G 9/087* (2006.01)

(86) International application number:
**PCT/JP2011/070901**

(87) International publication number:
**WO 2012/036171 (22.03.2012 Gazette 2012/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.09.2010 JP 2010206201**

(71) Applicant: **Hodogaya Chemical Co., Ltd.**
**Tokyo 104-0028 (JP)**

(72) Inventors:
- **KIMURA, Ikuo**
**Tsukuba-shi**
**Ibaraki 305-0841 (JP)**
- **ITO, Masami**
**Tsukuba-shi**
**Ibaraki 305-0841 (JP)**
- **TOJO, Masaya**
**Tsukuba-shi**
**Ibaraki 305-0841 (JP)**
- **ASAKAI, Masafumi**
**Tsukuba-shi**
**Ibaraki 305-0841 (JP)**

(74) Representative: **Müller-Boré & Partner**
**Patentanwälte**
**Grafinger Straße 2**
**81671 München (DE)**

# (54) CHARGE CONTROL AGENT AND TONER USING SAME

(57) To provide a charge controlling agent for electrophotography, which presents sufficient triboelectric chargeability to a toner, is useful particularly for a color toner and further for a polymerized toner, increases a charge rising rate, has a high electric charge amount, is excellent in charging characteristics, stability over time and environmental stability and yet is safe without posing any problem regarding waste regulations, and a negatively chargeable toner for developing an electrostatic image, which uses such a charge controlling agent and which has a high charging performance. A charge controlling agent comprising, as an effective component, at least one type of cyclic phenol sulfide represented by the following formula (1), and a toner comprising such a charge controlling agent:

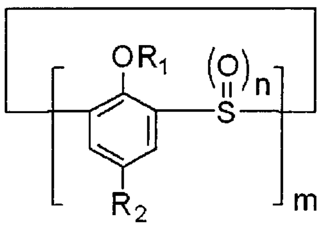

(1)

EP 2 618 220 A1

Fig. 1

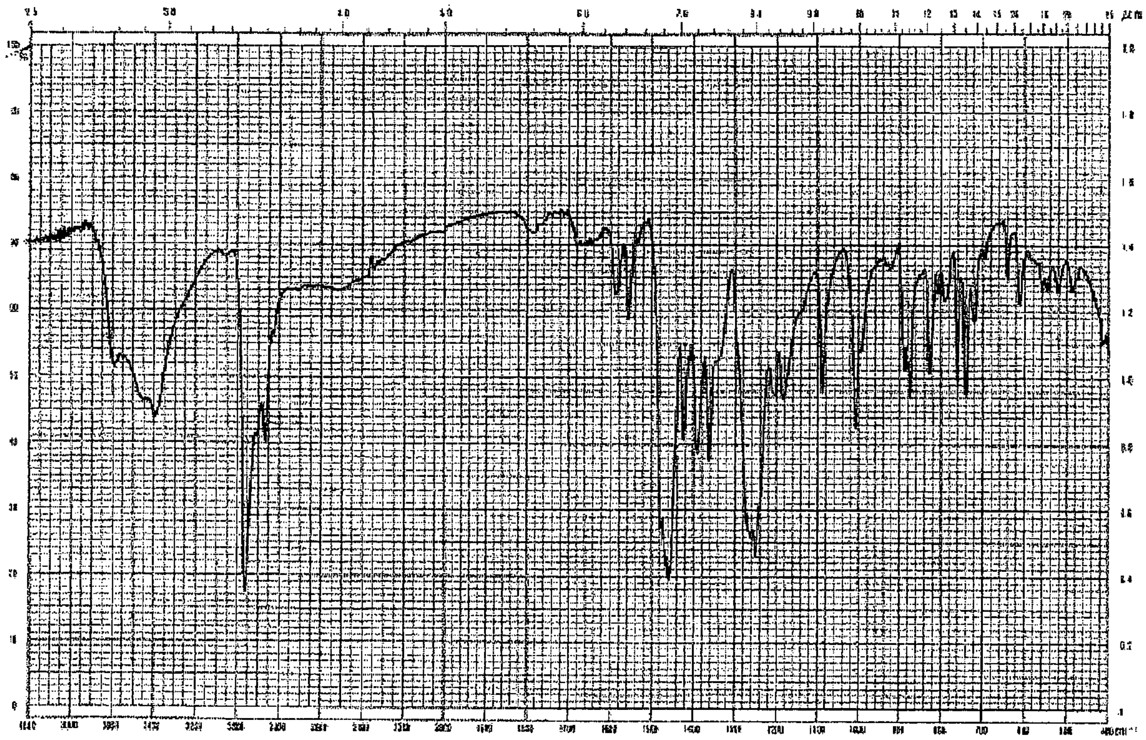

**Description**

TECHNICAL FIELD

The present invention relates to a charge controlling agent to be used in an image-forming apparatus for developing an electrostatic latent image in the field of electrophotography, electrostatic recording, etc., and a negatively chargeable toner containing such a charge controlling agent.

BACKGROUND ART

In an image-forming process by electrophotographic system, a visible image is obtained by forming an electrostatic latent image on an inorganic photosensitive material such as selenium, a selenium alloy, cadmium sulfide or amorphous silicon or on an organic photosensitive material using a charge generating agent and a charge transport agent, developing the electrostatic latent image with a toner, transferring the developed image onto paper or a plastic film, and fixing the transferred image thereon. The photosensitive material has a positive chargeability or a negative chargeability depending on its constitution, and when leaving an electrostatic image on a part to be printed by light exposure, development is carried out with a reversely charged toner. On the other hand, when carrying out reverse development by destaticizing a part to be printed, development is carried out with the same side charged toner.

A toner comprises a binder resin, a colorant and other additives. A charge controlling agent is generally added in order to provide satisfactory charging characteristics (including a charging speed, a charging level, a charging stability, etc.), stability over time, environmental stability, etc. Properties of the toner are substantially improved by addition of the charge controlling agent.

As a positively triboelectrically chargeable charge controlling agent known today in this technical field, a nigrosine dye, an azine dye, a copper phthalocyanine pigment, a quaternary ammonium salt or a polymer having a quaternary ammonium salt in its side chain may, for example, be mentioned. As a negatively triboelectrically chargeable charge controlling agent, a metal complex salt of a monoazo dye, a metal complex salt of salicylic acid, naphthoic acid or dicarboxylic acid, a copper phthalocyanine pigment, or a resin containing an acid component, is, for example, known.

Further, In the case of a color toner, the market of which is expected to become large in future, it is indispensable to use a pale color, preferably colorless, charge controlling agent which does not present an influence on hue. Examples of such a pale color or colorless charge controlling agent, for a negatively chargeable toner, include a metal complex salt compound of a hydroxybenzoic acid derivative (e.g. Patent Document 1), an aromatic dicarboxylic acid metal salt compound (e.g. Patent Document 2), a metal complex salt compound of an anthranilic acid derivative (e.g. Patent Document 3), an organic boron compound (e.g. Patent Document 4), a biphenol compound (e.g. Patent Document 5), a calyx(n)arene compound (e.g. Patent Document 6) and a cyclic phenol sulfide (e.g. Patent Documents 7 to 9). Further, for a positively chargeable toner, a quaternary ammonium salt compound (e.g. Patent Document 10) may, for example, be mentioned.

PRIOR ART DOCUMENTS

PATENT DOCUMENTS

Patent Document 1: JP-A-61-069073
Patent Document 2: JP-A-57-111541
Patent Document 3: JP-A-62-094856
Patent Document 4: U.S.P. 4,767,688
Patent Document 5: JP-A-61-003149
Patent Document 6: Japanese Patent No. 3,313,871
Patent Document 7: JP-A-2003-295522
Patent Document 8: W02007-111346
Patent Document 9: WO2007-119797
Patent Document 10: JP-A-58-009154
Patent Document 11: JP-A-10-081680
Patent Document 12: WO98/09959

NON-PATENT DOCUMENT

Non-patent Document 1: DENSHI SHASHIN GAKKAISHI (Electrophotography) Vol. 27, No. 3, p.307 (1988)

DISCLOSURE OF THE INVENTION

TECHNICAL PROBLEM

However, many of these charge controlling agents are complexes or salts made of a heavy metal such as chromium, and thus they are problematic with respect to waste regulations and cannot necessarily be said to be safe. Further, some of them cannot be made to be completely colorless, or are poor in the charge-imparting effect and inadequate in the charge-rising rate, whereby the initial copy image lacks in sharpness, or the quality of copy image tends to change during continuous copying, or the fluctuation range of the toner charging characteristics tends to be large against the environmental conditions such as the temperature, pressure, etc., whereby the image quality may substantially change due to e.g. season factors, and further, they may have a drawback that they cannot be applied to polymerized toners. Accordingly, a charge controlling agent has been desired which has a high charge-imparting effect and is applicable to polymerized toners.

SOLUTION TO PROBLEM

The present invention has been made to solve the above problem, and it is an object of the present invention to provide a charge controlling agent for electrophotography, which presents sufficient triboelectric chargeability to a toner, is useful particularly for a color toner and further for a polymerized toner, increases the charge rising rate, has a high electric charge amount, is excellent in charging characteristics, stability over time and environmental stability and yet is safe without posing any problem with respect to waste regulations. Further, it is another object of the present invention to provide a negatively chargeable toner for developing an electrostatic image, which uses such a charge controlling agent and which has a high charging performance.

The present invention has been made as a result of an extensive research to accomplish the above objects and provides the following.

1. A charge controlling agent comprising, as an effective component, at least one type of cyclic phenol sulfide represented by the following formula (1):

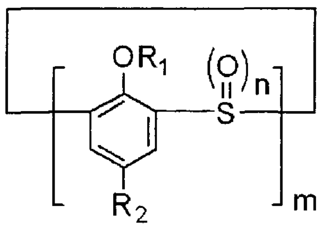

(1)

wherein $R_1$ is a hydrogen atom, a $C_{1-8}$ linear or branched alkyl group which may have a substituent, a substituted or unsubstituted aromatic hydrocarbon group, or a substituted or unsubstituted condensed polycyclic aromatic group, $R_2$ is a $C_{1-8}$ linear or branched alkyl group which may have a substituent, a substituted or unsubstituted aromatic hydrocarbon group, a substituted or unsubstituted aromatic heterocyclic group, or a substituted or unsubstituted condensed polycyclic aromatic group, m is an integer of from 4 to 9, and n is an integer of 0, 1 or 2, wherein the plurality of $R_1$ present in one molecule, may be the same or different from one another, provided that at least one of them is a hydrogen atom but not all of them are hydrogen atoms.

2. The charge controlling agent according to the above 1, wherein in the formula (1), n is 0.

3. The charge controlling agent according to the above 1 or 2, wherein in the formula (1), m is 4.

4. The charge controlling agent according to the above 2 or 3, which comprises, as an effective component, a mixture of three types of cyclic phenol sulfide of the formula (1), wherein m is 4, and n is 0.

5. The charge controlling agent according to any one of the above 1 to 4, wherein in the formula (1), $R_1$ is a hydrogen atom, or a $C_{1-4}$ linear or branched alkyl group which has no substituent.

6. The charge controlling agent according to any one of the above 1 to 5, wherein in the formula (1), $R_2$ is a $C_{1-4}$ linear or branched alkyl group which may have a substituent.

7. A toner comprising the charge controlling agent as defined in any one of the above 1 to 6, and a colorant and a

binder resin.

8. A polymerized toner comprising the charge controlling agent as defined in any one of the above 1 to 6, and a colorant and a binder resin.

In "a $C_{1-8}$ linear or branched alkyl group which may have a substituent" represented by $R_1$ or $R_2$ in the formula (1), the "$C_{1-8}$ linear or branched alkyl group" may specifically be a group such as a methyl group, an ethyl group, a n-propyl group, a 2-propyl group, a n-butyl group, a sec-butyl group, a 2-methylpropyl group, a tert-butyl group, a n-pentyl group, a 1-methylbutyl group, a 1-ethylpropyl group, a 1,1-dimethylpropyl group, a 1,2-dimethylpropyl group, a n-hexyl group, a 1-methylpentyl group, a 2-methylpentyl group, a 3-methylpentyl group, a 4-methylpentyl group, a 1-ethylbutyl group, a 2-ethylbutyl group, a 1,1-dimethylbutyl group, a 1,2-dimethylbutyl group, a 1,3-dimethylbutyl group, a 1,4-dimethylbutyl group, a 2,2-dimethylbutyl group, a 2,3-dimethylbutyl group, a 3,3-dimethylbutyl group, a 1-ethyl-2-methyl-propyl group, a 1,1,2-trimethylpropyl group, a n-heptyl group, a 2-methylhexyl group, a n-octyl group, an isooctyl group, a tert-octyl group, a 2-ethylhexyl group or a 3-methylheptyl group. Here, $R_1$ is preferably a $C_{1-4}$ linear or branched alkyl group which has no substituent, and $R_2$ is preferably a $C_{1-4}$ linear or branched alkyl group which may have a substituent.

In "a $C_{1-8}$ linear or branched alkyl group which may have a substituent" represented by $R_1$ or $R_2$ in the formula (1), the "substituent" may specifically be a group such as a deuterium atom, a fluorine atom, a chlorine atom, a cyano group, a hydroxy group, a nitro group, a cyclopentyl group, a cyclohexyl group, a $C_{1-6}$ linear or branched alkoxy group, a dialkylamino group substituted by a $C_{1-6}$ linear or branched alkyl group, a $C_{1-8}$ linear or branched acyl group, a $C_{1-8}$ linear or branched alkoxycarbonyl group, a $C_{1-6}$ epoxyalkyl group, a phenyl group, a naphthyl group, an anthryl group, a fluorenyl group, a styryl group, a pyridyl group, a pyridoindolyl group, a quinolyl group or a benzothiazolyl group. These substituents may further be substituted.

In "a substituted or unsubstituted aromatic hydrocarbon group" and "a substituted or unsubstituted condensed polycyclic aromatic group" represented by $R_1$ in the formula (1), the "aromatic hydrocarbon group" or the "condensed polycyclic aromatic group" may specifically be a group such as a phenyl group, a biphenylyl group, a terphenylyl group, a naphthyl group, an anthryl group, a phenanthryl group, a fluorenyl group, an indenyl group or a pyrenyl group.

In "a substituted aromatic hydrocarbon group" or "a substituted condensed polycyclic aromatic group" represented by $R_1$ in the formula (1), the "substituent" may specifically be a group such as a deuterium atom, a fluorine atom, a chlorine atom, a cyano group, a hydroxy group, a nitro group, a $C_{1-6}$ linear or branched alkyl group, a cyclopentyl group, a cyclohexyl group, a $C_{1-6}$ linear or branched alkoxy group, a dialkylamino group substituted by a $C_{1-6}$ linear or branched alkyl group, a phenyl group, a naphthyl group, an anthryl group, a fluorenyl group, a styryl group, a pyridyl group, a pyridoindolyl group, a quinolyl group or a benzothiazolyl group. These substituents may further be substituted.

In "a substituted or unsubstituted aromatic hydrocarbon group", "a substituted or unsubstituted aromatic heterocyclic group", or "a substituted or unsubstituted condensed polycyclic aromatic group" represented by $R_2$ in the formula (1), the "aromatic hydrocarbon group", the "aromatic heterocyclic group" or the "condensed polycyclic aromatic group" may specifically be a group such as a phenyl group, a biphenylyl group, a terphenylyl group, a naphthyl group, an anthryl group, a phenanthryl group, a fluorenyl group, an indenyl group, a pyrenyl group, a pyridyl group, a triazyl group, a pyrimidinyl group, a furanyl group, a pyranyl group, a thiophenyl group, a quinolyl group, an isoquinolyl group, a benzofuranyl group, a benzothiophenyl group, an indolyl group, a carbazolyl group, a benzoxazolyl group, a benzothiazolyl group, a quinoxalyl group, a benzoimidazolyl group, a pyrazolyl group, a dibenzofuranyl group, a dibenzothiophenyl group, a naphthyridinyl group, a phenanthroninyl group or an acridinyl group.

In "a substituted aromatic hydrocarbon group", "a substituted aromatic heterocyclic group", or "a substituted condensed polycyclic aromatic group" represented by $R_2$ in the formula (1), the "substituent" may specifically be a group such as a deuterium atom, a fluorine atom, a chlorine atom, a cyano group, a hydroxy group, a nitro group, a $C_{1-6}$ linear or branched alkyl group, a cyclopentyl group, a cyclohexyl group, a $C_{1-6}$ linear or branched alkoxy group, a dialkylamino group substituted by a $C_{1-6}$ linear or branched alkyl group, a phenyl group, a naphthyl group, an anthryl group, a fluorenyl group, a styryl group, a pyridyl group, a pyridoindolyl group, a quinolyl group or a benzothiazolyl group. These substituents may further be substituted.

In the formula (1), a plurality of n in each molecule may be the same or different, however, all n are preferably 0.

In the formula (1), m is preferably 4.

In the cyclic phenol sulfide represented by the formula (1) to be used in the present invention, the content of an alkali metal such as sodium in the product is at most 1,000 ppm. Accordingly, as compared with a toner containing, as an effective component, a conventional cyclic phenol sulfide wherein the content of an alkali metal such as sodium in the product exceeds 1,000 ppm, a toner containing, as an effective component, the cyclic phenol sulfide represented by the formula (1) wherein the content of an alkali metal such as sodium in the product is at most 1,000 ppm, has superiority such that it will be instantaneously electrostatically charged to a proper level (the time constant is small) and is excellent in environmental stability, particularly in that the electrostatic charge will not decrease even under a high temperature and high humidity condition.

As a factor to increase the content of an alkali metal such as sodium in the product, inclusion during the process

for production of an inorganic salt composed mainly of an alkali metal such as sodium is conceivable, and the content of an alkali metal such as sodium to be measured in the present invention is considered to represent a result including all of such a factor. The content of an alkali metal such as sodium can be measured by a usual measuring method, such as a fluorescent X-ray analysis, an atomic absorption analysis, an ICP emission analysis, an ICP-MS measurement or an analysis using ion chromatography, however, it is preferred to employ a fluorescent X-ray analysis from the viewpoint of practical efficiency.

A charge controlling agent may be defined to be one having a function to impart a stable static charge to a toner, but in a case where inorganic salts formed as reaction byproducts or unreacted organic salts are present in an amount larger than a certain level in the cyclic phenol sulfide, the influence of such salts becomes too large to ignore, and the stability of an image is likely to be disturbed in running for a long period of time not only in a high humidity environment but also in a normal humidity environment.

The measurement of salts in the charge controlling agent may be possible by measuring the electric conductivity as dispersed in water. However, in the case of organic salts, there may be a case where their solubility in water is so poor that it is not possible to obtain the content accurately.

In the present invention, it has been made possible to provide a charge controlling agent capable of exhibiting excellent charging performance and a toner using such a charge controlling agent, by directly measuring an alkali metal such as sodium contained in the cyclic phenol sulfide and controlling the content of an alkali metal such as sodium to be within a certain range.

The charge controlling agent to be used in the present invention is excellent in charge controlling characteristics, environmental resistance and durability, and when it is used for a pulverized toner or a polymerized toner, it is possible to obtain an image which is free from fogging and excellent in the image density, dot reproducibility and fine line reproducibility.

## ADVANTAGEOUS EFFECTS OF INVENTION

In the present invention, the charge controlling agent comprising, as an effective component, at least one type of cyclic phenol sulfide represented by the formula (1), has a higher charge rising rate than a conventional charge controlling agent, and it has a high electric charge amount and charging characteristics particularly excellent in environmental stability. Further, it does not contain a heavy metal such as chromium which is feared to present an environmental problem and further is excellent in the dispersibility and stability of the compound.

## BRIEF DESCRIPTION OF DRAWINGS

Fig. 1 is an IR chart of the compound in Example 1 of the present invention.
Fig. 2 is an IR chart of the compound in Example 5 of the present invention.

## DESCRIPTION OF EMBODIMENTS

The cyclic phenol sulfide represented by the formula (1) to be used in the present invention can be produced by subjecting a corresponding phenol derivative to a known cyclization reaction or such a cyclization reaction followed by an oxidation reaction (e.g. Patent Documents 11 and 12) to produce a corresponding cyclic phenol sulfide, and then, carrying out a known O-alkylation reaction or the like. Further, the cyclic phenol sulfide represented by the formula (1) to be used in the present invention can be produced by subjecting a corresponding cyclic phenol sulfide substituted by a halogen atom such as an iodine atom or a bromine atom, to a cross-coupling reaction such as Suzuki coupling.

As a method for reducing the content of an alkali metal such as sodium, a method may, for example, be mentioned wherein the cyclic phenol sulfide represented by the formula (1) produced as described above, is dissolved in an organic solvent, and an acid is added and stirred, followed by repeating washing with water. Here, the solvent to dissolve the cyclic phenol sulfide represented by the formula (1) may be any solvent so long as it is an organic solvent which is capable of dissolving the cyclic phenol sulfide and not miscible with water, however, an organic solvent having a high dissolving power and not miscible with water, such as 1,3-dimethyl-imidazolidinone or chloroform, is particularly preferred. Further, the acid to be added to the solution of the cyclic phenol sulfide in order to remove an alkali metal such as sodium, may be an inorganic acid or an organic acid so long as it is a strong acid, however, concentrated sulfuric acid or trifluoroacetic acid is particularly preferred.

In the present invention, it is preferred to adjust the volume average particle size of the charge controlling agent to be within a range of from 0.1 to 20 μm, particularly preferably within a range of from 0.1 to 10 μm. If the volume average particle size is less than 0.1 μm, the charge controlling agent appearing on the surface of the toner becomes

extremely brittle, whereby the desired charge controlling effect tends to be hardly obtainable, and if it exceeds 20 μm, the charge controlling agent falling off from the toner tends to increase, thus leading to an adverse effect such as contamination in the machine.

Further, when used for a polymerized toner of the present invention, it is preferred to adjust the volume average particle size to be at most 1.0 μm, particularly preferably within a range of from 0.01 to 1.0 μm. If the volume average particle size exceeds 1.0 μm, the particle size distribution of the finally obtainable electrophotographic toner tends to be wide, or formation of free particles is likely to occur, whereby the performance or reliability is likely to deteriorate. On the other hand, when the average particle size is within the above range, there will be no such drawbacks, and there will be such advantages that localization of the toner decreases, dispersion in the toner will be good, and fluctuation in the performance or reliability will be less.

As a method for incorporating the cyclic phenol sulfide represented by the formula (1) as the charge controlling agent to be used in the present invention, to the toner, there may be a method of preliminarily adding it to the inside of toner particles (internal addition) like a method of adding it together with a colorant, etc. to a binder resin, followed by kneading and pulverization (pulverized toner) or a method of adding the cyclic phenol sulfide represented by the formula (1) to a polymerizable monomer, followed by polymerization to obtain a toner (polymerized toner), or a method of preliminarily preparing toner particles and adding it to the surface of toner particles (external addition). In a case where it is internally added to the toner particles, the amount of the cyclic phenol sulfide to be added is preferably from 0.1 to 10 parts by mass, more preferably from 0.2 to 5 parts by mass, per 100 parts by mass of the binder resin. Further, in a case where it is externally added to the toner particles, its amount is preferably from 0.01 to 5 parts by mass, more preferably from 0.01 to 2 parts by mass, per 100 parts by mass of the binder resin. Further, it is preferred to fix it mechanochemically on the surface of the toner particles.

Further, in the present invention, the charge controlling agent comprising, as an effective component, the cyclic phenol sulfide represented by the formula (1), may be used in combination with known another negatively chargeable charge controlling agent. A preferred charge controlling agent to be used in combination may, for example, be an azo type iron complex or complex salt, an azo type chromium complex or complex salt, an azo type manganese complex or complex salt, an azo type cobalt complex or complex salt, an azo type zirconium complex or complex salt, a chromium complex or complex salt of a carboxylic acid derivative, a zinc complex or complex salt of a carboxylic acid derivative, an aluminum complex or complex salt of a carboxylic acid derivative, or a zirconium complex or complex salt of a carboxylic acid derivative. As the above carboxylic acid derivative, an aromatic hydroxy carboxylic acid is preferred, and 3,5-di-tert-butyl salicylic acid is more preferred. A preferred charge controlling agent to be used in combination, may further be a boron complex or complex salt, or a negatively chargeable resin type charge controlling agent.

In the present invention, in a case where the charge controlling agent is used in combination with another charge controlling agent, the amount of the charge controlling agent other than the charge controlling agent being the cyclic phenol sulfide represented by the formula (1) is preferably from 0.1 to 10 parts by mass per 100 parts by mass of the binder resin.

With respect to the type of the binder resin to be used for the toner of the present invention, the binder resin may be any known binder resin. It may, for example, be a vinyl polymer obtainable from e.g. a styrene type monomer, an acrylate type monomer or a methacrylate type monomer, or a copolymer obtainable from two or more types of such monomers, a polyester type monomer, a polyol resin, a phenol resin, a silicone resin, a polyurethane resin, a polyamide resin, a furan resin, an epoxy resin, a xylene resin, a terpene resin, a coumarone-indene resin, a polycarbonate resin or a petroleum type resin.

Now, the styrene type monomer, the acrylate type monomer and the methacrylate type monomer to be used for forming the above mentioned vinyl polymer or copolymer will be exemplified below, but they are not limited to such examples.

The styrene type monomer may, for example, be styrene or its derivative, such as styrene, o-methylstyrene, m-methylstyrene, p-methylstyrene, p-phenylstyrene, p-ethylstyrene, 2,4-dimethylstyrene, p-n-amylstyrene, p-tert-butylstyrene, p-n-hexylstyrene, p-n-octylstyrene, p-n-nonylstyrene, p-n-decylstyrene, p-n-dodecylstyrene, p-methoxystyrene, p-chlorostyrene, 3,4-dichlorostyrene, m-nitrostyrene, o-nitrostyrene or p-nitrostyrene.

The acrylate type monomer may, for example, be acrylic acid or its ester, such as acrylic acid, methyl acrylate, ethyl acrylate, propyl acrylate, n-butyl acrylate, isobutyl acrylate, n-octyl acrylate, n-dodecyl acrylate, 2-ethylhexyl acrylate, stearyl acrylate, 2-chloroethyl acrylate or phenyl acrylate.

The methacrylate type monomer may, for example, be methacrylic acid or its ester, such as methacrylic acid, methyl methacrylate, ethyl methacrylate, propyl methacrylate, n-butyl methacrylate, isobutyl methacrylate, n-octyl methacrylate, n-dodecyl methacrylate, 2-ethylhexyl methacrylate, stearyl methacrylate, phenyl methacrylate, dimethylaminoethyl methacrylate or diethylaminoethyl methacrylate.

Examples of other monomers to be used for forming the above vinyl polymer or copolymer include the following (1) to (18). (1) Monoolefins such as ethylene, propylene, butylene and isobutylene; (2) polyenes such as butadiene and isoprene; (3) vinyl halides such as vinyl chloride, vinylidene chloride, vinyl bromide and vinyl fluoride; (4) vinyl esters

such as vinyl acetate, vinyl propionate and vinyl benzoate; (5) vinyl ethers such as vinyl methyl ether, vinyl ethyl ether and vinyl isobutyl ether; (6) vinyl ketones such as vinyl methyl ketone, vinyl hexyl ketone and methyl isopropenyl ketone; (7) N-vinyl compounds such as N-vinyl pyrrole, N-vinyl carbazole, N-vinyl indole and N-vinyl pyrrolidone; (8) vinyl naphthalenes; (9) acrylic acid or methacrylic acid derivatives, such as acrylonitrile, methacrylonitrile and acrylamide; (10) unsaturated dibasic acids such as maleic acid, citraconic acid, itaconic acid, alkenyl succinic acid, fumaric acid and mesaconic acid; (11) unsaturated dibasic acid anhydrides such as maleic anhydride, citraconic anhydride, itaconic anhydride and alkenyl succinic anhydride; (12) monoesters of unsaturated dibasic acids, such as maleic acid monomethyl ester, maleic acid monoethyl ester, maleic acid monobutyl ester, citraconic acid monomethyl ester, citraconic acid monoethyl ester, citraconic acid monobutyl ester, itaconic acid monomethyl ester, alkenyl succinic acid monomethyl ester, fumaric acid monomethyl ester and mesaconic acid monomethyl ester; (13) unsaturated dibasic acid esters such as dimethyl maleate and dimethyl fumarate; (14) a-β-unsaturated acids such as crotonic acid and cinnamic acid; (15) α-β-unsaturated acid anhydrides such as crotonic anhydride and cinnamic anhydride; (16) carboxy group-containing monomers, such as an anhydride of such an α-β-unsaturated acid and a lower fatty acid, alkenyl malonic acid, alkenyl glutaric acid, alkenyl adipic acid, their acid anhydrides and their monoesters; (17) acrylic acid or methacrylic acid hydroxy alkyl esters, such as 2-hydroxyethyl acrylate, 2-hydroxyethyl methacrylate and 2-hydroxypropyl methacrylate; (18) hydroxy group-containing monomers, such as 4-(1-hydroxy-1-methylbutyl)styrene and 4-(1-hydroxy-1-methylhexyl)styrene.

In the toner of the present invention, the vinyl polymer or copolymer as the binder resin may have a crosslinking structure crosslinked by a crosslinking agent having at least two vinyl groups. The crosslinking agent to be used in such a case may, for example, be an aromatic divinyl compound such as divinyl benzene or divinyl naphthalene. Diacrylate compounds bonded with alkyl chains include, for example, ethylene glycol diacrylate, 1,3-butylene glycol diacrylate, 1,4-butanediol diacrylate, 1,5-pentanediol diacrylate, 1,6-hexanediol diacrylate and neopentyl glycol diacrylate, or ones having acrylate in the above compounds substituted by methacrylate.

The acrylate compounds bonded with alkyl chains containing ether bonds include, for example, diethylene glycol diacrylate, triethylene glycol diacrylate, tetraethylene glycol diacrylate, polyethylene glycol #400 diacrylate, polyethylene glycol #600 diacrylate and dipropylene glycol diacrylate, or ones having acrylate of the above compounds substituted by methacrylate.

Further, a diacrylate compound or a dimethacrylate compound, bonded with a chain containing an aromatic group and an ether bond, may also be mentioned. As a polyester type diacrylate, MANDA, tradename, (manufactured by NIPPON KAYAKU Co., Ltd.) may, for example, be mentioned.

A polyfunctional crosslinking agent includes, for example, pentaerythritol triacrylate, trimethylolethane triacrylate, trimethylolpropane triacrylate, tetramethylolmethane tetraacrylate, oligoester acrylate and ones having acrylate in the above compounds substituted by methacrylate, triallyl cyanurate, and triallyl trimellitate.

Such a crosslinking agent is used in an amount of preferably from 0.01 to 10 parts by mass, particularly preferably from 0.03 to 5 parts by mass, per 100 parts by mass of other monomer components. Among these crosslinkable monomers, an aromatic divinyl compound (particularly preferably divinyl benzene) and a diacrylate compound bonded with a chain containing one ether bond and an aromatic group may be mentioned as ones to be suitably used as resins for toners from the viewpoint of the fixing property and offset resistance. Among them, a combination of monomers to form a styrene type copolymer or a styrene/acrylate type copolymer, is preferred.

In the present invention, the polymerization initiator to be used for the preparation of the vinyl polymer or copolymer may, for example, be 2,2'-azobisisobutylonitrile, 2,2'-azobis(4-methoxy-2,4-dimethylvaleronitrile), 2,2'-azobis(-2,4-dimethylvaleronitrile), 2,2'-azobis(2-methylbutylonitrile), dimethyl-2,2'-azobisisobutyrate, 1,1'-azobis(1-cyclohexanecarbonitrile), 2-(carbamoylazo)-isobutylonitrile, 2,2'-azobis(2,4,4,-trimethylpentane), 2-phenylazo-2',4'-dimethyl-4'-methoxyvaleronitrile, 2,2'-azobis(2-methyl-propane); a ketone peroxide such as methyl ethyl ketone peroxide, acetylacetone peroxide or cyclohexanone peroxide; 2,2-bis(tert-butylperoxy)butane, tert-butyl hydroperoxide, cumene hydroperoxide, 1,1,3,3-tetramethylbutyl hydroperoxide, di-tert-butyl peroxide, tert-butylcumyl peroxide, dicumyl peroxide, α(tert-butylperoxy)isopropylbenzene, isobutyl peroxide, octanoyl peroxide, decanoyl peroxide, lauroyl peroxide, 3,5,5-trimethylhexanoyl peroxide, benzoyl peroxide, m-tolyl peroxide, di-isopropyl peroxydicarbonate, di-2-ethylhexyl peroxydicarbonate, di-n-propyl peroxydicarbonate, di-2-ethoxyethyl peroxycarbonate, di-ethoxyisopropyl peroxydicarbonate, bis(3-methyl-3-methoxybutyl) peroxycarbonate, acetylcyclohexylsulfonyl peroxide, tert-butyl peroxyacetate, tert-butylperoxyisobutylate, tert-butylperoxy-2-ethylhexalate, tert-butylperoxylaurate, tert-butyl-oxybenzoate, tert-butylperoxyisopropylcarbonate, di-tert-butylperoxyisophthalate, tert-butylperoxyarylcarbonate, isoamylperoxy-2-ethylhexanoate, di-tert-butylperoxyhexahydroterephthalate, or tert-butylperoxyazelate.

In a case where the binder resin is a styrene/acrylate type copolymer resin, a resin having at least one peak in a molecular weight region of from 3,000 to 50,000 (calculated as a number average molecular weight) and at least one peak in a molecular weight region of at least 100,000, in the molecular weight distribution by gel permeation chromatography (hereinafter referred to simply as GPC) of a soluble component in a tetrahydrofuran (hereinafter referred to simply as THF), of the copolymer resin, is preferred from the viewpoint of the fixing property, offset property and

storage stability. Further, a binder resin is also preferred in which in the THF soluble component, a component having a molecular weight distribution of at most 100,000 is from 50 to 90%. Further preferred is one having the main peak in a molecular weight region of from 5,000 to 30,000, most preferably in a molecular weight region of from 5,000 to 20,000.

The acid value of a vinyl polymer such as a styrene/acrylate type copolymer resin as the binder resin is preferably from 0.1 mgKOH/g to 100 mgKOH/g, more preferably from 0.1 mgKOH/g to 70 mgKOH/g, particularly preferably from 0.1 mgKOH/g to 50 mgKOH/g.

Monomers to constitute a polyester type polymer as a binder resin may be the following ones.

A dihydric alcohol component may, for example, be ethylene glycol, propylene glycol, 1,3-butanediol, 1,4-butanediol, 2,3-butanediol, diethylene glycol, triethylene glycol, 1,5-pentanediol, 1,6-hexanediol, neopentyl glycol, 2-ethyl-1,3-hexanediol, hydrogenated bisphenol A, or a diol obtainable by polymerization of a cyclic ether such as ethylene oxide or propylene oxide to bisphenol A.

It is preferred to use a trihydric or higher hydric alcohol in combination in order to crosslink a polyester resin. Such a trihydric or higher polyhydric alcohol may, for example, be sorbitol, 1,2,3,6-hexanetetrol, 1,4-sorbitan, pentaerythritol, dipentaerythritol, tripentaerythritol, 1,2,4-butanetriol, 1,2,5-pentatriol, glycerol, 2-methylpropanetriol, 2-methyl-1,2,4-butanetriol, trimethylolethane, trimethylolpropane or 1,3,5-trihydroxybenzene.

Examples of an acid component to form the above polyester type polymer, include benzene dicarboxylic acids such as phthalic acid, isophthalic acid and terephthalic acid or their anhydrides; alkyl dicarboxylic acids such as succinic acid, adipic acid, sebacic acid and azelaic acid or their anhydrides; unsaturated dibasic acids such as maleic acid, citraconic acid, itaconic acid, alkenyl succinic acid, fumaric acid and mesaconic acid; and unsaturated dibasic acid anhydrides such as maleic anhydride, citraconic anhydride, itaconic anhydride and an alkenyl succinic anhydride. Also, examples of a trivalent or higher polycarboxylic acid component include trimellitic acid, pyromellitic acid, 2,5,7-naphthalenetricarboxylic acid, 1,2,4-naphthalenetricarboxylic acid, 1,2,4-butanetricarboxylic acid, 1,2,5-hexanetricarboxylic acid, 1,3-dicarboxyl-2-methyl-2-methylenecarboxypropane, tetra(methylenecarboxyl)methane, 1,2,7,8-octanetetracarboxylic acid and embole trimer acid, or their anhydrides and their partial lower alkyl esters.

In a case where the binder resin is a polyester type resin, it is preferred that at least one peak is present in a molecular weight region of from 3,000 to 50,000 in the molecular weight distribution of the THF-soluble component of the resin component, from the viewpoint of the fixing property and the offset resistance of the toner. Further, a binder resin is also preferred in which in the THF-soluble component, a component having a molecular weight of at most 100,000 is from 60 to 100%. Further, preferred is one wherein at least one peak is present within the molecular weight region of from 5,000 to 20,000.

In the present invention, the molecular weight distribution of the binder resin is measured by GPC using THF as a solvent.

In a case where the binder resin is a polyester resin, its acid value is preferably from 0.1 mgKOH/g to 100 mgKOH/g, more preferably from 0.1 mgKOH/g to 70 mgKOH/g, particularly preferably from 0.1 mgKOH/g to 50 mgKOH/g.

Further, its hydroxy value is preferably at most 30 mgKOH/g, more preferably from 10 mgKOH/g to 25 mgKOH/g.

In the present invention, one or more non-crystalline polyester resins and one or more crystalline polyester resins may be used as mixed. In such a case, it is preferred to select the materials taking into the respective compatibilities into consideration.

A non-crystalline polyester resin is preferably one prepared from a polyvalent carboxylic acid component, preferably an aromatic polyvalent carboxylic acid, and a polyhydric alcohol component.

A crystalline polyester resin is preferably one prepared from a dicarboxylic acid component, preferably an aliphatic dicarboxylic acid, and a dihydric component.

As a binder resin useful for the toner of the present invention, it is also possible to use such a resin that the above mentioned vinyl polymer component and/or polyester type resin component contains a monomer component reactive with both of such resin components. Among monomers to constitute the polyester type resin component, one reactive with the vinyl polymer may, for example, be an unsaturated dicarboxylic acid such as phthalic acid, maleic acid, citraconic acid or itaconic acid, or its anhydride. A monomer to constitute the vinyl polymer component may be one having a carboxy group or a hydroxy group, or an acrylic acid or methacrylic acid ester.

In a case where the polyester type polymer, the vinyl polymer and another binder resin are used in combination, it is preferred that resins having an acid value of from 0.1 to 50 mgKOH/g constitute at least 60 mass% based on the entire binder resins.

In the present invention, the acid value of the binder resin component in the toner composition is obtained by the following method, and the basic operation is in accordance with JIS K-0070.

(1) As a sample, one having additives other than the binder resin (polymer component) preliminarily removed, is used, or the acid values and contents of components other than the binder resin and the crosslinked binder resin are preliminarily obtained. A pulverized product of the sample is accurately weighed in an amount of from 0.5 to 2.0 g, and the weight of the polymer component is designated as Wg. For example, in a case where the acid value of

the binder resin is to be measured from the toner, the acid values and contents of the colorant, magnetic substance, etc. may be separately measured, and the acid value of the binder resin may be obtained by calculation.

(2) The sample is put into a 300 ml beaker, and 150 ml of a mixed liquid of toluene/ethanol (volume ratio: 4/1) is added for dissolution.

(3) Using an ethanol solution containing 0.1 mol/L of KOH, titration is carried out by means of a potentiometric titration apparatus.

(4) The amount of the KOH solution used at that time is designated as S (ml), and at the same time, a blank is measured and the amount of the KOH solution used at that time is designated as B (ml), whereupon calculation is made by the following formula (1), wherein f is a factor of the KOH concentration.

$$\text{Acid value (mgKOH/g)} = [(S-B) \times f \times 5.61]/W \qquad (1)$$

The binder resin for a toner and the composition containing such a binder resin preferably has a glass transition temperature (Tg) of from 35 to 80°C, particularly preferably from 40 to 75°C, from the viewpoint of the resistibility of the toner. If Tg is lower than 35°C, the toner tends to deteriorate in a high temperature atmosphere, or offset is likely to occur during the fixing. Further, if Tg exceeds 80°C, the fixing property tends to decrease.

In a polymerized toner of the present invention, a binder resin having a softening point within a range of from 80 to 140°C is preferably employed. If the softening point of the binder resin is lower than 80°C, the toner and the image stability of the toner are likely to deteriorate after the fixing or during the storage. On the other hand, if the softening point exceeds 140°C, the low temperature fixing property is likely to deteriorate.

The toner of the present invention comprising a charge controlling agent containing, as an effective component, at least one type of cyclic phenol sulfide represented by the formula (1), and a colorant and a binder resin, may further contain a magnetic material, so that it will be a magnetic toner.

The magnetic material useful for the toner of the present invention may, for example, be (1) a magnetic iron oxide such as magnetite, maghemite or ferrite, or an iron oxide containing another metal oxide, (2) a metal such as iron, cobalt or nickel, or an alloy of such a metal with another metal such as aluminum, cobalt, copper, lead, magnesium, tin, zinc, antimony, beryllium, bismuth, cadomium, calcium, manganese, selenium, titanium, tungsten or vanadium, or (3) a mixture thereof.

Specific examples of the magnetic material include $Fe_3O_4$, $\gamma$-$Fe_2O_3$, $ZnFe_2O_4$, $Y_3Fe_5O_{12}$, $CdFe_2O_4$, $Gd_3Fe_5O_{12}$, $CuFe_2O_4$, $PbFe_{12}O$, $NiFe_2O_4$, $NdFe_2O$, $BaFe_{12}O_{19}$, $MgFe_2O_4$, $MnFe_2O_4$, $LaFeO_3$, iron powder, cobalt powder and nickel powder. The above-mentioned magnetic materials may be used alone, or in combination as a mixture of two or more of them. A particularly preferred magnetic material is a fine powder of triiron tetraoxide or $\gamma$-diiron trioxide.

Further, a magnetic iron oxide such as magnetite, maghemite or ferrite, containing a different element, or a mixture of such magnetic iron oxides, may be used. The different element may, for example, be lithium, beryllium, boron, magnesium, aluminum, silicon, phosphorous, germanium, zirconium, tin, sulfur, calcium, scandium, titanium, vanadium, chromium, manganese, cobalt, nickel, copper, zinc or gallium. A preferred different element is selected from magnesium, aluminum, silicon, phosphorous and zirconium. The different element may be taken into the iron oxide crystal lattice, may be taken, in the form of an oxide, in the iron oxide, or may be present in the form of an oxide or hydroxide on the surface. However, it is preferably contained in the form of an oxide.

The above-mentioned different element may be taken into particles by letting a salt of such a different element be present and adjusting the pH at the time of forming a magnetic material. Otherwise, it may be precipitated on the surface of particles by adjusting the pH after forming magnetic material particles, or by adding salts of the respective elements, followed by adjusting the pH.

The amount of the magnetic material to be used is from 10 to 200 parts by mass, preferably from 20 to 150 parts by mass, of the magnetic material, per 100 parts by mass of the binder resin. Such a magnetic material preferably has a number average particle size of from 0.1 to 2 $\mu$m, more preferably from 0.1 to 0.5 $\mu$m. The number average particle size can be obtained by measuring, e.g. by a digitizer, an enlarged photograph taken by a transmission electron microscope.

Further, with respect to the magnetic properties, the magnetic material is preferably one having, as magnetic properties under application of 10 K oersted, a coercive force of 20 to 150 oersted, a saturation magnetization of from 50 to 200 emu/g and a residual magnetization of from 2 to 20 emu/g.

The above magnetic material may be used also as a colorant. As a colorant useful in the present invention, in the case of a black toner, black or blue dye or pigment particles may be mentioned. The black or blue pigment may, for example, be carbon black, aniline black, acetylene black, phthalocyanine blue or indanthrene blue. The black or blue dye may, for example, be an azo type dye, an anthraquinone type dye, a xanthene type dye or a methine type dye.

When used for a color toner, the colorant may be the following. A magenta colorant may, for example, be a

condensed azo compound, a diketo-pyrrolo-pyrrole compound, an anthraquinone compound, a quinacridone compound, a basic dye, a lake dye, a naphthol dye, a benzimidazolone compound, a thioindigo compound or a perylene compound. Specifically, a pigment type magenta colorant includes C.I. Pigment Red 1, 2, 3, 4, 5, 6, 7, 8, 9, 10, 11, 12, 13, 14, 15, 16, 17, 18, 19, 21, 22, 23, 30, 31, 32, 37, 38, 39, 40, 41, 48, 49, 50, 51, 52, 53, 54, 55, 57, 58, 60, 63, 64, 68, 81, 83, 87, 88, 89, 90, 112, 114, 122, 123, 163, 202, 206, 207, 209, Pigment Violent 19, C.I. Bat Red 1, 2, 10, 13, 15, 23, 29, and 35, etc.

The above pigment may be used alone, however, from the viewpoint of the image quality of a full color image, it is more preferred to use a pigment and a dye in combination to improve the sharpness.

A dye type magenta colorant includes oil-soluble dyes such as C.I. Solvent Red 1, 3, 8, 23, 24, 25, 27, 30, 49, 81, 82, 83, 84, 100, 109, 121, C.I. Disperse Red 9, C.I. Solvent Violent 8, 13, 14, 21 and 27, and C.I. Disperse Violet 1, and basic dyes such as C.I. Basic Red 1, 2, 9, 12, 13, 14, 15, 17, 18, 22, 23, 24, 27, 29, 32, 34, 35, 36, 37, 38, 39 and 40, and C.I. Basic Violet 1, 3, 7, 10, 14, 15, 21, 25, 26, 27 and 28.

As a cyan colorant, a copper phthalocyanine compound or its derivative, anthraquinone or a basic dye lake compound may be used. Specifically, a pigment type cyan colorant may, for example, be C.I. Pigment Blue 2, 3, 15, 16, 17, C.I. Bat Blue 6, C.I. Acid Blue 45, or a copper phthalocyanine pigment having from 1 to 5 phthalimide metal groups substituted on its phthalocyanine skeleton.

As a yellow colorant, a condensed azo compound, an isoindolinone compound, an anthraquinone compound, an azo metal complex, a methine compound or an allylamide compound may be used. Specifically, a yellow pigment includes C.I. Pigment Yellow 1, 2, 3, 4, 5, 6, 7, 10, 11, 12, 13, 14, 15, 16, 17, 23, 65, 73 and 83, and C.I. Bat Yellow 1, 3 and 20, etc.

An orange pigment may, for example, be reddish chrome yellow, molybdenum orange, permanent orange GTR, pyrazolone orange, vulcan orange, benzidine orange G, indanthrene brilliant orange RK or indanthrene brilliant orange GK. A purple pigment may, for example, be manganese violet, fast violet B or methyl violet lake. A green pigment may, for example, be chromium oxide, chrome green, pigment green, malachite green lake or final yellow green G. A white pigment may, for example, be zinc oxide, titanium oxide, antimony white or zinc sulfide.

The amount of such a colorant is preferably from 0.1 to 20 parts by mass per 100 parts by mass of the binder resin.

The toner of the present invention may be mixed with a carrier and used as a two-component developer. The carrier to be used in the present invention may be a usual carrier such as ferrite of magnetite, or a resin-coated carrier may also be used.

The resin-coated carrier is composed of carrier core particles and a covering material being a resin to cover (coat) the surface of carrier core particles, and the resin to be used for such a covering material is preferably a styrene-acrylate resin such as a styrene/acrylate copolymer or a styrene/methacrylate copolymer, an acrylate resin such as an acrylate copolymer or a methacrylate copolymer, a fluorinated resin such as a polytetrafluoroethylene, a monochlorotrifluoroethylene polymer or a polyvinylidene fluoride, a silicone resin, a polyester resin, a polyamino resin, a polyvinyl butyral or an amino acrylate resin. It may further be a resin which can be used as a material to cover (coat) a carrier, such as an iomonomer resin or a polyphenylene sulfide resin. These resins may be used alone, or in combination as a mixture of a plurality of them.

Further, it is also possible to use binder type carrier cores having magnetic powder dispersed in a resin. As a method for covering the surface of carrier cores with at least a resin covering agent for a resin-coated carrier, it is possible to use a method wherein a resin is dissolved or suspended in a solvent and applied to deposit on the carrier cores, or a method wherein they are simply mixed in a powder state. The proportion of the resin covering material to the resin-coated carrier may suitably be decided, but it is preferably from 0.01 to 5 mass%, more preferably from 0.1 to 1 mass%, to the resin-coated carrier.

A practical example to cover a magnetic material with a covering (coating) agent made of a mixture of two or more types, may, for example, be (1) one having 100 parts by mass of fine powder of titanium oxide is treated with 12 parts by mass of a mixture of dimethyl dichlorosilane and dimethyl silicone oil (mass ratio of 1:5) or (2) one having 100 parts by mass of fine powder of silica treated with 20 parts by mass of a mixture of dimethyl dichlorosilane or dimethyl silicone oil (mass ratio of 1:5).

Among the above resins, a styrene/methyl methacrylate copolymer, a mixture of a fluorinated resin and a styrene type copolymer, or a silicone resin, is preferably employed, and a silicone resin is particularly preferred.

The mixture of a fluorinated resin and a styrene type copolymer may, for example, be a mixture of a polyvinylidene fluoride and a styrene/methyl methacrylate copolymer, a mixture of a polytetrafluoroethylene and a styrene/methyl methacrylate copolymer, or a mixture of a vinylidene fluoride/tetrafluoroethylene copolymer (copolymer mass ratio of from 10:90 to 90:10), a styrene/2-ethylhexyl acrylate copolymer (copolymer mass ratio of from 10:90 to 90:10) and a styrene/2-ethylhexyl acrylate/methyl methacrylate copolymer (copolymer mass ratio of from 20 to 60: from 5 to 30:10:50).

The silicone resin may, for example, be a nitrogen-containing silicone resin, or a modified silicone resin formed by reacting a silicone resin with a nitrogen-containing silane coupling agent.

As the magnetic material for carrier cores, an oxide such as ferrite, iron-excessive type ferrite, magnetite or γ-iron oxide, a metal such as iron, cobalt or nickel, or an alloy thereof may be used. Further, elements contained in these magnetic materials may, for example, be iron, cobalt, nickel, aluminum, copper, lead, magnesium, tin, zinc, antimony, beryllium, bismuth, calcium, manganese, selenium, titanium, tungsten and vanadium. Preferred one may, for example, be copper-zinc-iron type ferrite containing copper, zinc and iron components as the main components, or manganese-magnesium-iron type ferrite containing manganese, magnesium and iron components as the main components.

The resistance value of the carrier is preferably adjusted to be from $10^6$ to $10^{10}$ $\Omega \cdot$ cm by adjusting the surface roughness of the carrier or the amount of the resin to be coated. The particle size of the carrier may be from 4 to 200 $\mu$m, preferably from 10 to 150 $\mu$m, more preferably from 20 to 100 $\mu$m. Particularly, the resin-coated carrier preferably has a 50% particle size of from 20 to 70 $\mu$m.

In a two-component developer, it is preferred to use the toner of the present invention in an amount of from 1 to 200 parts by mass per 100 parts by mass of the carrier, and more preferred to use the toner in an amount of from 2 to 50 parts by mass per 100 parts by mass of the carrier.

The toner of the present invention may further contain a wax. The wax to be used in the present invention may be the following. It may, for example, be an aliphatic hydrocarbon type wax, such as a low molecular weight polyethylene, a low molecular weight polypropylene, a polyolefin wax, microcrystalline wax, paraffin wax or sasol wax, an oxide of such an aliphatic hydrocarbon type wax, such as an oxidized polyethylene wax, a block copolymer thereof, a plant wax such as candelilla wax, carnauba wax, Japanese wax or jojoba wax, an animal-based wax such as beeswax, lanolin or spermaceti wax, a mineral wax such as ozokerite, ceresin or petrolatum, a wax composed mainly of a fatty acid ester, such as montanic acid ester wax or castor wax, or one having a part or whole of the fatty acid ester deoxidized, such as deoxidized carnauba wax.

Examples of the wax further include a saturated straight chain fatty acid such as palmitic acid, stearic acid, montanic acid or a linear alkyl carboxylic acid having a linear alkyl group; an unsaturated fatty acid plandinic acid, eleostearic acid, or parinaric acid; a saturated alcohol such as stearyl alcohol, eicosyl alcohol, behenyl alcohol, carnaubil alcohol, glyceryl alcohol, melissyl alcohol or a long chain alkyl alcohol; a polyhydric alcohol such as sorbitol; a fatty acid amide such as linoleic acid amide, olefinic acid amide or lauric acid amide; a saturated fatty acid bisamide such as methylene biscaprylic acid amide, ethylene bislauric acid amide or hexamethylene bisstearic acid amide; an unsaturated fatty acid amide such as ethylene bisoleic acid amide, hexamethylene bisoleic acid amide, N,N'-dioleyl adipic acid amide or N,N'-dioleyl sebacic acid amide; an aromatic bisamide such as m-xylene bisstearic acid amide or N,N'-distearyl isophthalic acid amide; a fatty acid metal salt such as calcium stearate, calcium laurate, zinc stearate or magnesium stearate; a wax having a vinyl type monomer such as styrene or acrylate grafted to an aliphatic hydrocarbon type wax; a partially esterified compound of a fatty acid with a polyhydric alcohol, such as biphenic acid monoglyceride; and a methylester compound having a hydroxy group, obtainable by hydrogenating a plant oil or fat.

A wax which may be preferably used, includes a polyolefin obtained by radical polymerization of an olefin under a high pressure; a polyolefin obtained by purifying a low molecular weight by-product obtainable at the time of polymerization for a high molecular weight polyolefin; a polyolefin obtained by polymerization by means of a catalyst such as a Ziegler catalyst or a metallocene catalyst under a low pressure; a polyolefin obtained by polymerization utilizing a radiation, electromagnetic waves or light; a low molecular weight polyolefin obtainable by thermal decomposition of a high molecular weight polyolefin; paraffin wax, microcrystalline wax or Fischer-Tropsch wax; a synthetic hydrocarbon wax prepared by a Synthol method, a Hydrochol method or an Arge method; a synthetic wax obtained by using as a monomer a compound having one carbon atom; a hydrocarbon type wax having a functional group such as a hydroxy group or a carboxy group; a mixture of a hydrocarbon type wax and a hydrocarbon type wax having a functional group; and a wax obtained by graft-modifying the above wax as a matrix with a vinyl monomer such as xylene, a maleic acid ester, an acrylate, a methacrylate or maleic anhydride.

Further, these waxes having their molecular weight distribution sharpened by a press sweating process, a solvent method, a recrystallization method, a vacuum distillation method, a super critical gas extraction method or a solution crystallization method, or ones having a low molecular weight solid fatty acid, a low molecular weight solid alcohol, a low molecular weight solid compound or other impurities removed, may preferably be used.

The wax to be used in the present invention preferably has a melting point of from 50 to 140°C, more preferably from 70 to 120°C, in order to take a balance of the fixing property and the offset resistance. If it is less than 50°C, the blocking resistance tends to be low, and if it exceeds 140°C, the offset resistance effect tends to be hardly obtainable.

Further, by using at least two different types of waxes in combination, it is possible to simultaneously obtain a plasticizing action and a releasing action being actions of the waxes.

A type of wax having a plasticizing action may, for example, be a wax having a low melting point, or one having a branch in its molecular structure or one with a structure having a polar group. Whereas, a wax having a releasing action may, for example, be a wax having a high melting point, or one having a straight chain structure as its molecular structure or one with no polarity having no functional group. As a practical example, a combination of at least two different types of waxes with a difference in the melting points being from 10°C to 100°C, or a combination of a polyolefin and a graft-modified polyolefin, may, for example, be mentioned.

In a case where two types of waxes are selected for use, in the case of waxes having similar structures, a wax having a relatively low melting point exhibits a plasticizing action, and a wax having a relatively high melting point exhibits a releasing action. At that time, the difference in melting point is preferably from 10 to 100°C, since the functional separation is thereby effectively be obtainable. When it is less than 10°C, no adequate effect for the functional separation tends to be obtainable, and if it exceeds 100°C, the functional collaboration by the mutual actions tends to be hardly obtainable. In such a case, the melting point of at least one wax is preferably from 70 to 120°C, further preferably from 70 to 100°C, whereby the effect for functional separation tends to be easily obtainable.

Further, waxes are relatively such that one having a branched structure, one having polar group such as a functional group, or one modified with a component different from the main component, exhibits a plasticizing action, while one having a more straight chain structure, one with no polarity having no functional group, or a non-modified straight structured one, exhibits a releasing action. A preferred combination may, for example, be a combination of a polyethylene homopolymer or copolymer composed essentially of ethylene, with a polyolefin homopolymer or copolymer composed mainly of an olefin other than ethylene; a combination of a polyolefin with a graft-modified polyolefin; a combination of an alcohol wax, a fatty acid wax or an ester wax, with a hydrocarbon type wax; a combination of Fischer-Tropsch wax or a polyolefin wax, with a paraffin wax or a microcrystalline wax; a combination of Fischer-Tropsch wax with a polyolefin wax; a combination of a paraffin wax with microcrystalline wax; or a combination of carnauba wax, candelilla wax, rice wax or montan wax, with a hydrocarbon type wax.

In each case, it is preferred that in the endothermic peaks observed in DSC measurement of a toner, the peak top temperature of the maximum peak is present in a region of from 70 to 110°C, further preferably the maximum peak is present in a region of from 70 to 110°C, whereby it becomes easy to take a balance between the fixing property and the storage stability of the toner.

In the toner of the present invention, the total content of such waxes is preferably from 0.2 to 20 parts by mass, more preferably from 0.5 to 10 parts by mass, per 100 parts by mass of the binder resin, from the viewpoint of the effectiveness.

In the present invention, the peak top temperature of the maximum peak among endothermic peaks of a wax measured in the DSC measurement, is taken as the melting point of the wax.

In the present invention, DSC measurement of a wax or a toner is carried out preferably by a high precision internally-heated input compensation type differential scanning calorimeter, the measuring method is carried out in accordance with ASTM D3418-82. The DSC curve to be used in the present invention is such that after taking a preliminary history by raising and lowering the temperature once, the temperature is raised at a rate of 10°C/min, and the DSC curve measured at that time is employed.

The toner of the present invention may contain a flowability-improving agent. The flowability-improving agent is one to improve the flowability of a toner (to make it readily flowable) by adding it to the surface of the toner. It may, for example, be a fluorinated resin powder such as vinylidene fluoride fine powder or polytetrafluoroethylene fine powder, a silica fine powder such as wet process-produced silica or dry process-produced silica, a titanium oxide fine powder, or a treated silica, a treated titanium or a treated alumina, which is surface-treated with a silane coupling agent, a titanium coupling agent or a silicone oil. Among them, a silica fine powder, a titanium oxide fine powder or an alumina fine powder is preferred, and a treated silica which is surface-treated with a silane coupling agent or a silicone oil is further preferred. The particle size of the flowability-improving agent is preferably from 0.001 to 2 μm, particularly preferably from 0.002 to 0.2 μm, as the average primary particle size.

A preferred silica fine powder is a fine powder formed by vapor phase oxidation of a silicone halide compound, which is so-called dry process-produced silica or filmed silica.

As commercially available silica fine powders formed by vapor phase oxidation of silicone halide compounds, those commercially available under the following tradenames are, for example, available; AEROSIL (manufactured by Nippon Aerosil Co., Ltd., the same applies hereinafter) -130, -300, -380, -TT600, -MOX170, -MOX80 or -COK84: Ca-O-SiL (manufactured by CABOT Corporation, the same applies hereinafter) -M-5, -MS-7, -MS-75, -HS-5 or -EH-5; Wacker HDK (manufactured by WACKER-CHEMIE GMBH, the same applies hereinafter) -N20 V15, -N20E, -T30 or -T40; D-CFinesilica (manufactured by Dow Corning Corporation); and Fransol (manufactured by Fransil Company).

Further, the silica fine powder formed by vapor phase oxidation of a silicone halide compound is further subjected to hydrophobic treatment to obtain a treated silica fine powder, which is more preferred. The treated silica fine powder is particularly preferably one obtained by treating a silica fine powder so that the hydrophobicity measured by a methanol titration test preferably shows a value of from 30 to 80%. The hydrophobicity is imparted by chemically or physically treating the silica fine powder with e.g. an organic silicone compound which is reactive or physically absorptive with the silica fine powder. A preferred method is a method wherein a silica fine powder formed by vapor phase oxidation of a silicone halide compound is treated with an organic silicone compound.

The organic silicone compound may, for example, be hydroxypropyl trimethoxysilane, phenyl trimethoxysilane, n-hexadecyl trimethoxysilane, n-octadecyl trimethoxysilane, vinyl methoxysilane, vinyl triethoxysilane, vinyl triacetoxysilane, dimethylvinyl chlorosilane, divinyl chlorosilane, γ-methacryloxypropyl trimethoxysilane, hexamethyl disilane,

trimethylsilane, trimethyl chlorosilane, dimethyl dichlorosilane, methyltrichlorosilane, allyldimethyl chlorosilane, allylphenyl dichlorosilane, benzyldimethyl chlorosilane, bromomethyldimethyl chlorosilane, α-chloroethyl trichlorosilane, vapor-chloroethyl trichlorosilane, chloromethyl dimethyl chlorosilane, triorganosilyl mercaptan, trimethylsilyl mercaptan, triorganosilyl acrylate, vinyl dimethylacetoxysilane, dimethylethoxysilane, trimethylethoxysilane, trimethylmethoxysilane, isobutyltrimethoxysilane, dimethyldimethoxysilane, diphenyldiethoxysilane, hexamethyldisiloxane, 1,3-divinyl tetramethyldisiloxane, 1,3-diphenyltetramethyldisiloxane, and a dimethylpolysiloxane having from 2 to 12 siloxane units per molecule and also having from 0 to 1 hydroxy group bonded to Si in a unit located at the terminal. Further, a silicone oil such as dimethyl silicone oil may be mentioned. They may be used alone respectively, or in combination as a mixture of two or more of them.

The flowability-improving agent is preferably one having a number average particle size of from 5 to 100 nm, more preferably from 5 to 50 nm. One having a specific surface area of preferably at least 30 $m^2/g$, more preferably from 60 to 400 $m^2/g$, by nitrogen adsorption measured by a BET method, is preferred, and as the surface-treated fine powder, the specific surface area is preferably at least 20 $m^2/g$, particularly preferably from 40 to 300 $m^2/g$. the amount of such fine powder to be applied is preferably from 0.03 to 8 parts by mass, per 100 parts by mass of the toner particles.

The toner of the present invention may further contain other additives such as various metal soaps, a fluorinated surfactant, dioctyl phthalate, etc. in order to protect a photosensitive material and a carrier, to improve a cleaning property, to adjust thermal, electric or physical properties, to adjust a resistance, to adjust a softening point, to improve a fixing rate, etc., and may further include an electroconductivity-imparting agent such as tin oxide, zinc oxide, carbon black, antimony oxide, etc. and inorganic fine powders such as titanium oxide, aluminum oxide, alumina, etc. Also, these inorganic fine powders may be optionally subjected to hydrophobic treatment. Also, the toner may further contain a lubricant such as polytetrafluoroethylene, zinc stearate, or vinylidene polyfluoride, an abradant such as cesium oxide, silicon carbide or strontium titanate, an anti-caking agent, and a development-improving agent such as black fine particles and white fine particles having a polarity reverse to the toner particles in a small amount.

In order to control a charging amount, these additives are preferably treated with various treating agents including a silicone varnish, various modified silicone varnishes, a silicone oil, various modified silicone oils, a silane coupling agent, a silane coupling agent having a functional group, and other organic silicone compounds.

In the present invention, the charge controlling agent is, together with the above-mentioned additives and toner, fully mixed and stirred by a mixer such as a Henschel mixer, a ball mill, a nauta mixer, a V-type mixer, a W-type mixer or a super mixer to have the surface of toner particles uniformly externally treated thereby to obtain a desired electrostatic image developing toner.

The toner of the present invention is thermally stable and can retain a stable chargeability without being susceptive to a thermal change in an electrophotographic process. Also, since it is uniformly dispersed in any binder resin, a charge distribution of a fresh toner becomes very uniform, and the toner of the present invention including untransferred and recovered toner (used toner) does not show a substantial change in a saturated tribo-charged amount and a charge distribution as compared with a fresh toner. When a used toner resulting from the electrostatic image developing toner of the present invention is to be reused, it is possible to further make a difference between the fresh toner and the used toner smaller by preparing a toner using a polyester resin including an aliphatic diol as a binder resin or a metal-crosslinked styrene-acrylate copolymer as a binder resin and also using a large amount of polyolefin added thereto.

As a method for producing the toner of the present invention, it may be produced by a known production method. As an example of the production method, a method (pulverization method) is preferred wherein the above-described materials to constitute a toner, such as the binder resin, the charge controlling agent, the colorant, etc. are sufficiently mixed by a mixer such as a ball mill, and the mixture is kneaded well by a heat-kneading apparatus such as a hot roll kneader, then cooled for solidification, pulverized and then classified.

Otherwise, it may be produced also by a method of dissolving the above mixture in a solvent, followed by spraying to form fine particles which are then dried and classified. Further, it may be produced by a method for producing a toner by a polymerization method wherein predetermined materials are mixed to a monomer to constitute a binder resin to form an emulsion or suspension, which is then polymerized to obtain a toner, or a method wherein in a so-called microcapsule toner made of a core material and a shell material, predetermined materials are incorporated to the core material or the shell material or to both of them. Further, the toner of the present invention may be produced by sufficiently mixing desired additives and toner particles, as the case requires, by a mixer such as a Henschel mixer.

The method for producing the toner of the present invention by the above-mentioned pulverization method will be described in further detail. Firstly, the binder resin, the colorant, the charge controlling agent and other necessary additives are uniformly mixed. The mixing may be carried out by using a known mixer such as a Henschel mixer, a super mixer or a ball mill. The obtained mixture is heat-melted and kneaded by means of a closed system kneader or a single screw or twin screw extruder. The kneaded product is cooled and then roughly pulverized by means of a crusher or a hammer mill and further finely pulverized by a pulverizer such as a jet mill or a high speed rotation mill. Further, classification is carried out to a predetermined particle size by means of a wind force classification device, for example, an elbow jet of inertial classification system utilizing the Coanda effect, a microplex of cyclone (centrifugal) classification

system or a DS separator. Further, in a case where the surface of a toner is to be treated with an external additive, the toner and the external additive are mixed and stirred by a high speed mixer such as a Henschel mixer or a super mixer.

Further, the toner of the present invention may be produced also by a suspension polymerization method or an emulsion polymerization method. In the suspension polymerization method, a polymerizable monomer, a colorant, a polymerization initiator, a charge controlling agent, and further, as the case requires, a crosslinking agent, a dispersion stabilizer and other additives, are uniformly dissolved or dispersed to prepare a monomer composition, and then, this monomer composition is dispersed in a continuous phase containing a dispersion stabilizer, e.g. a water phase, by means of a suitable mixing or dispersing machine such as a homomixer, a homogenizer, an atomizer, a microfluidizer, a one liquid-fluid nozzle, a gas-liquid-fluid nozzle or an electric emulsifier. Preferably, granulation is carried out by adjusting the mixing speed, temperature and time so that liquid droplets of the polymerizable monomer composition have the desired toner particle size. At the same time, the polymerization reaction is carried out at a temperature of from 40 to 90°C to obtain toner particles having a desired particle size. The obtained toner particles are washed, subjected to filtration and then dried. After the production of toner particles, treatment with an external additive may be carried out by the above-described method.

When produced by an emulsion polymerization method, the toner particles are excellent in the uniformity as compared with particles obtained by the above-described suspension polymerization method, but the average particle size is very small at a level of from 0.1 to 1.0 μm, and therefore, in some cases, it is possible to produce the toner by a so-called seed polymerization to let particles grow by post adding a polymerizable monomer to emulsified particles as nuclei, or by a method to combine or fuse emulsified particles to a suitable average particle size.

The production by these polymerization methods does not involve a pulverization step, and therefore it is not required to impart brittleness to toner particles. Further, a low softening point material which used to be difficult to use in the conventional pulverization method, can be used in a large amount, and therefore the range for selection of the material can be broadened. A releasing agent or colorant made of a hydrophobic material is scarcely exposed on the surface of toner particles, whereby contamination to a toner carrier component, a photosensible material, a transfer roller or a fixing device can be reduced.

By producing the toner of the present invention by such a polymerization method, it is possible to further improve the properties such as image reproducibility, transfer properties and color reproducibility, and it is possible to reduce the particle size of the toner in order to meet with fine dots and to obtain a toner having a sharp particle size distribution relatively easily.

As a polymerizable monomer to be used at the time of producing the toner of the present invention by a polymerization method, a radical-polymerizable vinyl type polymerizable monomer is employed. As such a vinyl type polymerizable monomer, a monofunctional polymerizable monomer or a polyfunctional polymerizable monomer may be used.

The monofunctional polymerizable monomer may, for example, be a styrene type polymerizable monomer such as styrene, α-methylstyrene, β-methylstyrene, o-methylstyrene, m-methylstyrene, p-methylstyrene, 2,4-dimethylstyrene, p-n-butylstyrene, p-tert-butylstyrene, p-n-hexylstyrene or p-phenylstyrene; an acrylate type polymerizable monomer such as methyl acrylate, ethyl acrylate, n-propyl acrylate, isopropyl acrylate, n-butyl acrylate, isobutyl acrylate, tert-butyl acrylate, n-amyl acrylate, n-hexyl acrylate, 2-ethylhexyl acrylate, n-octyl acrylate, benzyl acrylate, dimethylphosphate methyl acrylate, dibutylphosphate ethyl acrylate or 2-benzoyloxyethyl acrylate; a methacrylate type polymerizable monomer such as methyl methacrylate, ethyl methacrylate, n-propyl methacrylate, isopropyl methacrylate, n-butyl methacrylate, isobutyl methacrylate, tert-butyl methacrylate, n-amyl methacrylate, n-hexyl methacrylate, 2-ethylhexyl methacrylate, n-octyl methacrylate, diethylphosphate methacrylate or dibutylphosphate ethyl methacrylate; an unsaturated aliphatic monocarboxylate; a vinyl ester such as vinyl acetate, vinyl propionate or vinyl benzoate; a vinyl ether such as vinyl methyl ether or vinyl isobutyl ether; or a vinyl ketone such as vinyl methyl ketone, vinyl hexyl ketone or vinyl isopropyl ketone.

As a polymerization initiator to be used at the time of producing the toner of the present invention by a polymerization method, a known initiator such as an organic peroxide may be used. A water-soluble initiator may, for example, be antimony persulfate, potassium persulfate, 2,2'-azobis(N,N'-dimethylene isobutyloamidine) hydrochloride, 2,2'-azobis (2-aminodipropane) hydrochloride, azobis(isobutyloamidine) hydrochloride, sodium 2,2'-azobisisobutyronitrile sulfonate, ferrous sulfate or hydrogen peroxide.

The polymerization initiator is used preferably in an amount of from 0.5 to 20 parts by mass per 100 parts by mass of the polymerizable monomer, and polymerization initiators may be used alone or in combination. With respect to a dispersing agent to be used at the time of producing the polymerized toner, for example, an inorganic type oxide may, for example, be tricalcium phosphate, magnesium phosphate, aluminum phosphate, zinc phosphate, calcium carbonate, magnesium carbonate, aluminum hydroxide, calcium metasilicate, calcium sulfate, barium sulfate, bentonite, silica or alumina. An organic compound may, for example, be polyvinyl alcohol, gelatin, methyl cellulose, methylhydroxypropyl cellulose, ethyl cellulose, a sodium salt of carboxymethyl cellulose or starch. Such a dispersing agent is used preferably in an amount of from 0.2 to 2.0 parts by mass per 100 parts by mass of the polymerizable monomer.

As such a dispersing agent, a commercial product may be used as it is. However, in order to obtain dispersed particles having a fine uniform particle size, the above inorganic compound may be formed under stirring at a high speed in a dispersing medium.

The toner obtainable by the polymerization method is protean and tends to have small degree of surface roughness of toner particles, as compared with a toner by the pulverization method which is not subjected to any special treatment, whereby the contact area of the electrostatic latent image support and the toner increases, and the toner adhesion tends to be high, and consequently, contamination in the machine decreases, and an image having a higher image concentration and higher quality tends to be readily obtainable.

Further, also for the toner by the pulverization method, a method for reducing the degree of surface roughness of the toner may be mentioned, such as a hot bath method of dispersing toner particles in water, followed by heating, a heat treatment method of permitting toner particles to pass through a hot air stream or a mechanical impact method of applying a mechanical energy to the toner particles for treatment. An apparatus effective to reduce the degree of surface roughness may, for example, be a mechanofusion system (manufactured by Hosokawa Micron Corporation) employing a dry-system mechanochemical method, I-type jet mill, Hybridizer (manufactured by Nara Machinery Co., Ltd.) being a mixing apparatus having a rotor and a liner, or a Henschel mixer as a mixer having a high speed stirring vanes.

An average degree of circularity may be used as one of values showing the degree of surface roughness of toner particles. The average degree of circularity (C) means a value obtained by obtaining a degree of circularity (Ci) by the following formula (2) and further dividing the sum of degrees of circularity of all particles measured as shown by the following formula (3) by the total number (m) of measured particles.

$$\text{Degree of circularity (Ci)} = \frac{\text{Perimeter of circle having the same projected area as particle}}{\text{Perimeter of projected image of particle}} \quad (2)$$

$$\text{Average degree of circularity} \quad C = \sum_{i=1}^{m} Ci / m \quad (3)$$

The above degree of circularity (Ci) is measured by means of a flow type particle image analyzer (e.g. FPIA-1000, manufactured by TOA Medical Electronics Co., Ltd.). The measuring method comprises preparing a dispersion having about 5 mg of a toner dispersed in 10 ml of water having about 0.1 mg of a nonionic surfactant dissolved therein, irradiating the dispersion with ultrasonic waves (20 kHz, 50 W) for 5 minutes to bring the dispersion concentration to be from 5,000 to 20,000 particles/μL, and measuring the circularity distribution of particles having circle-equivalent diameters of at least 0.60 μm and less than 159.21 μm by means of the above-mentioned flow type particle image measuring apparatus.

It is preferred to adjust the toner particles so that the value of the above average degree of circularity is preferably from 0.955 to 0.995, further preferably from 0.960 to 0.985, whereby a phenomenon to bring about an increase of the toner remaining after transfer tends to be small, and retransfer is less likely to occur.

In the case of the toner of the present invention, from the viewpoint of the imaging property and the productivity of the toner, for example, in the measurement using a laser type particle size distribution measuring apparatus such as a micronsizer (e.g. manufactured by Seishin Enterprise Co., Ltd.), in the case of a pulverized toner, the particle size of the toner is preferably within a range of from 2 to 15 μm, more preferably within a range of from 3 to 12 μm by volume-based average particle size. If the average particle size exceeds 15 μm, the resolution or the sharpness tends to deteriorate, and if the average particle size is less than 2 μm, although the resolution may be good, the yield in the production of the toner tends to deteriorate, thus leading to a problem of an increase of cost, or scattering of the toner in the machine or a health trouble such as skin penetration is likely to result.

On the other hand, in the case of a polymerized toner, the average particle size is preferably within a range of from 3 to 9 μm, more preferably within a range of from 4 to 8.5 μm, particularly preferably within a range of from 5 to 8 μm. If the volume average particle size is smaller than 3 μm, the flowability of the toner tends to decrease, and the chargeability of each particle tends to decrease, or the charge distribution tends to be broad, whereby fogging on the background or spilling of the toner from the developer is likely to occur. Further, if it is less than 3 μm, the cleaning tends to be substantially difficult. If the volume average particle size exceeds 9 μm, the resolution tends to decrease, and no adequate image quality tends to be obtainable, whereby it may become difficult to satisfy the requirement for high image

quality in recent years.

Further, when cumulative distributions of the volume and number, respectively, from the small size side are drawn for particle size ranges (channels) obtained by dividing a particle size distribution measured by the following method, and the particle size at cumulative 16% is defined as volume D16%, the particle size at cumulative 50% as volume D50% and the particle size at cumulative 84% as volume D84%, the polymerized toner of the present invention has a volume average particle size distribution index (GSDv) of preferably from 1.15 to 1.30, more preferably from 1.15 to 1.25, as calculated from (D84%/D16%) 1/2.

With regard to a particle size distribution of the toner of the present invention, by measuring a particle size by a COULTER COUNTER (TA-II manufactured by COULTER Co.), a content of particles having a particle size of at most 2 μm is preferably from 10 to 90% on the basis of the number of particles, and a content of particles having a particle size of at least 12.7 μm is preferably from 0 to 30% on the basis of volume.

Further, one having a high particle size uniformity (volume average particle size/number average particle size being from 1.00 to 1.30) is preferred.

The electrostatic image developing toner of the present invention preferably has a specific surface area of from 1.2 to 5.0 $m^2/g$, more preferably from 1.5 to 3.0 $m^2/g$, as measured by BET specific surface area measurement using nitrogen as a desorption-adsorption gas. The measurement of the specific surface area is carried out by using e.g. a BET specific surface area measuring apparatus (e.g. Flow Sorb II2300, manufactured by Shimadzu Corporation), and a specific surface area is defined as a value determined from a desorbed gas amount measured by desorbing a gas adsorbed on a toner surface at 50°C for 30 minutes, adsorbing a nitrogen gas again by rapidly cooling with liquid nitrogen, and heating to 50°C again for carrying out desorption again.

An apparent specific gravity (bulk density) of the toner of the present invention is measured by using e.g. a powder tester (manufactured by e.g. Hosokawa Micron Corporation). When the toner of the present invention is a non-magnetic toner, the toner should preferably have an apparent specific gravity of from 0.2 to 0.6 $g/cm^3$, and when the toner of the present invention is a magnetic toner, the toner should preferably have an apparent specific gravity of from 0.2 to 2.0 $g/cm^3$ although it may vary depending on a content and a type of a magnetic powder used.

When the toner of the present invention is a non-magnetic toner, a toner should preferably have a true specific gravity of from 0.9 to 1.2 $g/cm^3$, and when the toner is a magnetic toner, the toner should preferably have a true specific gravity of from 0.9 to 4.0 $g/cm^3$ although it varies depending on a content and a type of a magnetic powder used. The true specific gravity of the toner is measured by accurately measuring a weight of 1.000 g of the toner, placing the measured toner in a 10 mmΦ tablet-molding machine, press-molding under a pressure of 200 $kgf/cm^2$ in vacuum, and measuring a height of the molded product of cylindrical shape by a micrometer, thereby calculating a true specific gravity.

A flowability of a toner is defined by a flow angle of repose and a static angle of repose measured by e.g. a repose angle-measuring apparatus (manufactured by e.g. Tsutsui Rika K.K.). The electrostatic image developing toner using a charge controlling agent of the present invention preferably has a flow angle of repose of from 5° to 45° and a static angle of repose of from 10° to 50°.

In the case of a pulverized type toner, the toner of the present invention should preferably have an average value of shape coefficient (SF-1) of from 100 to 400 and an average value of shape coefficient (SF-2) of from 100 to 350.

In the present invention, shape coefficients SF-1 and SF-2 of a toner are calculated by sampling a group of about 30 toner particles as an image enlarged 1,000 times in one view by an optical microscope (such as BH-2, manufactured by Olympus Optical Co., Ltd.) equipped with a CCD camera, transferring obtained images to an image analyzing apparatus (such as Luzex FS manufactured by Nireco Corporation), and repeating the same operation until measuring about 1,000 toner particles. Shape coefficients SF-1 and SF-2 are calculated by the following formulae:

$$SF\text{-}1=((ML^2\times \pi)/4A)\times 100$$

(In the above formula, ML is a maximum length of a particle and A is a projected area of one particle.)

$$SF\text{-}2=(PM^2/4A\pi)\times 100$$

(In the above formula, PM is a circumference length of a particle and A is a projected area of one particle.)

SF-1 expresses a strain of a particle, and if a particle becomes closer to a sphere, the SF-1 value becomes closer to 100. And if a particle becomes longer and narrower, this value becomes larger. On the other hand, SF-2 expresses an irregularity degree of a particle surface, and if a particle becomes closer to a sphere, the SF-2 value becomes closer to 100, and if a particle shape becomes more complicated, the SF-2 value becomes larger.

The toner of the present invention preferably has a volume resistivity of from $1 \times 10^{12}$ to $1 \times 10^{16}$ Ω·cm in the

case of a non-magnetic toner and has a volume resistivity of from 1 × $10^8$ to 1 × $10^{16}$ Ω·cm in the case of a magnetic toner although it varies depending on a content and a type of a magnetic powder used. The volume resistivity of the toner is measured by pressure-molding toner particles into a disk-like test piece having a diameter of 50 mm and a thickness of 2 mm, fixing the test piece on an electrode for solid (e.g. SE-70 manufactured by Ando Electric Co., Ltd.), and measuring a resistance value one hour after continuously applying a direct current voltage of 100 V by using a high insulating resistance meter (e.g. 4339A manufactured by Hewlett-Packard Company).

The toner of the present invention preferably has a dielectric dissipation factor of from 1.0 × $10^{-3}$ to 15.0 × $10^{-3}$ in the case of a non-magnetic toner and has a dielectric dissipation factor of from 2 × $10^{-3}$ to 30 × $10^{-3}$ in the case of a magnetic toner although it varies depending on a content and a kind of a magnetic powder used. The dielectric dissipation factor of the toner is a dielectric dissipation factor (Tan δ) value obtainable by pressure-molding toner particles into a disk-like test piece having a diameter of 50 mm and a thickness of 2 mm, fixing the test piece on an electrode for solid, and measuring it at a frequency of 1 KHz and a peak to peak voltage of 0.1 KV by using a LCR meter (e.g. 4284A manufactured by Hewlett-Packard Company).

The toner of the present invention preferably has an Izod impact strength of from 0.1 to 30 kg·cm/cm. The Izod impact strength of the toner is measured by subjecting a plate-like test piece prepared by heat-melting toner particles to a test of JIS standard K-7110 (impact strength test method of rigid plastic).

The toner of the present invention preferably has a melt index (MI value) of from 10 to 150 g/10 min. The melt index (MI value) of the toner is one measured in accordance with JIS standard K-721 0 (A method). In this case, the temperature for the measurement is 125°C, and the load is 10 kg.

The toner of the present invention preferably has a melting-initiating temperature of from 80 to 180°C, and preferably has a 4 mm-descending temperature of from 90 to 220°C. The melt-initiating temperature of the toner is measured by pressure-molding toner particles into a cylindrical test piece having a diameter of 10 mm and a thickness of 20 mm, setting the test piece in a heat-melting property-measuring apparatus, e.g. a flow tester (e.g. CFT-500C manufactured by Shimadzu Corporation) and measuring a temperature value, at which a piston begins to descend under a load of 20 kgf/$cm^2$ at the initiation of melting. The 4 mm descending temperature of the toner is a temperature value, at which a piston descends 4 mm in the same test as above.

The toner of the present invention preferably has a glass transition temperature (Tg) of from 35 to 80°C, more preferably of from 40 to 75°C. The glass transition temperature of the toner is measured from a peak value in a phase change appearing when raising a temperature at a constant rate, rapidly cooling and raising a temperature again by using a differential thermogravimetry apparatus (hereinafter simply referred to as DSC). If the Tg value of the toner is lower than 35°C, an offset resistance and a storage stability tend to become poor and if the Tg value exceeds 80°C, a fixing strength of an image tends to be lowered.

Among endothermic peaks observed in DSC measurement of the toner of the present invention, a peak top temperature of the maximum peak is preferably present in a region of from 70 to 120°C.

The toner of the present invention preferably has a melt viscosity of from 1,000 to 50,000 poises, more preferably from 1,500 to 38,000 poises. The melt viscosity of the toner is measured by pressure-molding toner particles into a cylindrical test piece having a diameter of 10 mm and a thickness of 20 mm, setting the test piece in a heat melt property-measuring apparatus, e.g. a flow tester (CFT-500C manufactured by Shimadzu Corporation), and measuring the melt viscosity under a load of 20 kgf/$cm^2$.

A solvent-dissolved remaining content of the toner of the present invention is preferably from 0 to 30 mass% as a content insoluble in tetrahydrofuran (THF), from 0 to 40 mass% as a content insoluble in ethyl acetate and from 0 to 30 mass% as a content insoluble in chloroform. The solvent-dissolved remaining content is measured by uniformly dissolving or dispersing 1 g of a toner respectively in 100 ml of tetrahydrofuran (THF), ethyl acetate and chloroform, pressure-filtrating the solution or the dispersion, drying the filtrate to carry out quantitative determination, and calculating a percentage of an insoluble material of the toner, which is insoluble in the organic solvent.

The toner of the present invention can be used in a one-component developing system as one of image-forming methods. The one-component developing system is a system for developing a latent image by supplying a thin-filmed toner to a latent image support. The thin filming of the toner is carried out usually by means of an apparatus which comprises a toner-transporting member, a toner layer thickness-regulating member and a toner-supplementing member, wherein the toner supplementing member and the toner transporting member abut each other, and the toner layer thickness-regulating member and the toner transporting member abut each other.

A two-component developing system of using the toner of the present invention will be described in detail. The two-component developing system employs a toner and a carrier (having functions as a charge-imparting material and a toner-conveying material), and as the carrier, the above-mentioned magnetic material, or glass beads may be used. By stirring a developer (a toner and a carrier) by a stirring member, a predetermined charge amount is generated and is conveyed by e.g. a magnet roller to a site where development is carried out. By a magnetic force of the magnet roller, the developer is retained on the surface of the roller, and the developer is formed into a layer of appropriate height restricted by a developer-restricting plate to form a magnetic brush. The developer moves on the roller in accordance

with rotation of the developing roller in a contact state with an electrostatic latent image-holding material or in a non-contact state at a predetermined distance so as to be faced to the electrostatic latent image-holding material, and the latent image is developed into a visible image. In the development in the non-contact state, it is usual to produce a direct current electric field between the developer and the latent image-holding material, thereby providing a driving force for flying the toner between a predetermined distance space, or an alternating current field may also be applied in order to make a clearer image.

Further, the charge controlling agent of the present invention is suitable also as a charge controlling agent (charge-enhancing agent) for an electrostatic powder paint material. Thus, the electrostatic powder paint material using this charge-enhancing agent is excellent in environmental resistance, storage stability, particularly thermostability and durability, and a paint deposition efficiency reaches 100%, and a thick film having no painting defect can be formed.

## EXAMPLES

Hereinafter, the present invention is further described with reference to Examples and Comparative Examples, but is by no means limited thereto. In the following Examples and Comparative Examples, the term "part" means "part by mass".

## EXAMPLE 1

Analyses of the purity, compositional ratio, etc. of cyclic phenol sulfide represented by the formula (1) to be used in the present invention were carried out by high performance liquid chromatograph (hereinafter referred to simply as HPLC) measurements. The HPLC measurement conditions for the analysis of the compositional ratio in the obtained mixture of cyclic phenol sulfides are as follows. Apparatus LC-10A manufactured by Shimadzu Corporation, column: Develosil ODS-HG-5 (inner diameter: 4.6, column length: 250 mm) manufactured by Nomura Chemical Co., Ltd., column temperature: 40°C, mobile phase: THF/methanol/water/trifluoroacetic acid = 450/400/150/2 (v/v/v/v), flow rate: 1.0 ml/min, wavelength for measurement: 296 nm, injected amount: 1 μL, concentration of sample: 1,000 ppm.

Purification of such compounds was carried out by e.g. purification by column chromatograph, adsorption purification by e.g. silica gel, activated carbon or activated white earth, or recrystallization or precipitation method by means of a solvent. Further, identification of compounds was carried out by an IR analysis.

[Preparation Example 1]

Into a 1 L four-necked flask equipped with a stirrer, a condenser and a thermometer, 72.1 g of cyclic phenol sulfide corresponding to a cyclic tetramer of the formula (1) wherein all $R_1$ are hydrogen atoms, all $R_2$ are tert-butyl groups, m is 4 and n is 0, 700 ml of dimethylformamide (hereinafter referred to simply as DMF) and 55.3 g of potassium carbonate were added and heated to from 50°C to 52°C and then stirred for 3 hours. Then, 28.4 g of iodomethane was dropwise added, followed by further stirring at from 53°C to 54°C for 4 hours. The system was left to cool to room temperature, and insolubles were removed by filtration, followed by concentration under reduced pressure, and the concentrate was put into water. 0.5 ml of concentrated hydrochloric acid was added, followed by stirring. Precipitated crystals were collected by filtration, and then dispersion and washing with water and subsequent dispersion and washing with methanol, were repeated, followed by drying to obtain 45.4 g of a mixture of cyclic tetramers of the formula (1) wherein all $R_2$ are tert-butyl groups, m is 4, n is 0, and $R_1$ is a hydrogen atom or a methyl group, as whitish crystals.

With respect to the obtained whitish crystals, the structure was identified by means of IR. The results of the IR measurement are shown in Fig. 1.

Further, as a result of the HPLC analysis, the crystals were a mixture having such a compositional ratio that a product of the formula (1) wherein all $R_2$ are tert-butyl group, m is 4, n is 0 and one of $R_1$ is a methyl group and three of them are hydrogen atoms, was 2.0%, one wherein two $R_1$ are methyl groups, and other two $R_1$ are hydrogen atoms, was 83.9%, and one wherein three $R_1$ are methyl groups and one $R_1$ is a hydrogen atom, was 14.1%.

## EXAMPLE 2

[Preparation Example 2]

Into a 1 L four-necked flask equipped with a stirrer, a condenser and a thermometer, 72.1 g of cyclic phenol sulfide corresponding to a cyclic tetramer of the formula (1) wherein all $R_1$ are hydrogen atoms, all $R_2$ are tert-butyl groups, m is 4 and n is 0, 700 ml of DMF and 55.3 g of potassium carbonate were added, heated to from 28°C to 29°C and then stirred for two hours. Then, 28.4 g of iodomethane was dropwise added, followed by further stirring at from 27°C to 29°C for 4 hours. The system was left to cool to room temperature, and insolubles were removed by filtration,

followed by concentration under reduced pressure, and the concentrate was added to water. 0.5 ml of concentrated hydrochloric acid was added, followed by stirring. Precipitated crystals were collected by filtration, and then, dispersion and washing with water and subsequent dispersion and washing with methanol, were repeated, followed by drying to obtain 45.4 g of a mixture of cyclic tetramer of the formula (1) wherein all $R_2$ are tert-butyl groups, m is 4, n is 0, and $R_1$ is a hydrogen atom or a methyl group, as whitish crystals.

With respect to the obtained whitish crystals, the structure was identified by means of IR.

Further, from a result of the HPLC analysis, the crystals were a mixture having such a compositional ratio that the product of the formula (1) wherein all $R_2$ are tert-butyl groups, m is 4, n is 0, and one $R_1$ is methyl groups, and three $R_1$ are hydrogen atoms, was 11.7%, one wherein two $R_1$ are methyl groups, and other two $R_1$ are hydrogen atoms, was 76.6%, and one wherein three $R_1$ are methyl groups, and one $R_1$ is a hydrogen atom, was 11.7%.

EXAMPLE 3

[Preparation Example 3]

Into a 1 L four-necked flask equipped with a stirrer, a condenser and a thermometer, 72.1 g of cyclic phenol sulfide corresponding to a cyclic tetramer of the formula (1) wherein all $R_1$ are hydrogen atoms, all $R_2$ are tert-butyl groups, m is 4 and n is 0, 700 ml of DMF and 55.3 g of potassium carbonate were added, heated to from 50°C to 52°C and then stirred for 3 hours. Then, 28.4 g of iodomethane was dropwise added, followed by further stirring from 53°C to 54°C for 4 hours. The system was left to cool to room temperature, and insolubles were removed by filtration, followed by concentration under reduced pressure, and the concentrate was added to water. 0.5 ml of concentrated hydrochloric acid was added, followed by stirring. Precipitated crystals were collected by filtration and then, dispersion and washing with water and subsequent dispersion and washing with methanol, were repeated, followed by drying. Obtained crystals were dissolved in 500 ml of 1,3-dimethyl-2-imidazolidinone, and then, 40 ml of concentrated sulfuric acid was added, followed by stirring for 10 minutes. This solution was added to 2,000 ml of water with stirring. Precipitated crystals were collected by filtration, and then, dispersion and washing with water were carried out, followed by drying to reduce the content of an alkali metal such as sodium and to obtain 44.0 g of a mixture of cyclic tetramers of the formula (1) wherein all $R_2$ are tert-butyl groups, m is 4, n is 0, and $R_1$ is a hydrogen atom or a methyl group, as whitish crystals.

With respect to the obtained whitish crystals, the structure was identified by means of IR. Further, as a result of the HPLC analysis, the crystals were a mixture having such a compositional ratio that a product of the formula (1) wherein all $R_2$ are tert-butyl groups, m is 4, n is 0, and one $R_1$ is a methyl group, and three $R_1$ are hydrogen atoms, was 2.0%, one wherein two $R_1$ are methyl groups, and other two $R_1$ are hydrogen atoms, was 83.4%, and one wherein three $R_1$ are methyl groups, and one $R_1$ is a hydrogen atom, was 14.2%.

EXAMPLE 4

[Preparation Example 4]

Into a 1 L four-necked flask equipped with a stirrer, a condenser and a thermometer, 72.1 g of cyclic phenol sulfide corresponding to a cyclic tetramer of the formula (1) wherein all $R_1$ are hydrogen atoms, all $R_2$ are tert-butyl groups, m is 4 and n is 0, 700 ml of DMF and 55.3 g of potassium carbonate were added, heated to from 28°C to 29°C and then stirred for two hours. Then, 28.4 g of iodomethane was dropwise added, followed by further stirring at from 27°C to 29°C for 4 hours. The system was left to cool to room temperature, and insolubles were removed by filtration, followed by concentration under reduced pressure, and the concentrate was added to water. Then, 0.5 ml of concentrated hydrochloric acid was added, followed by stirring. Precipitated crystals were collected by filtration, and then, dispersion and washing with water were carried out, followed by drying to obtain 44.1 g of a mixture of cyclic tetramers of the formula (1) wherein all $R_2$ are tert-butyl groups, m is 4, n is 0, and $R_1$ is a hydrogen atom or a methyl group, as whitish crystals, having the content of an alkali metal such as sodium reduced.

With respect to the obtained whitish crystals, the structure was identified by means of IR. Further, as a result of the HPLC analysis, the crystals were a mixture having such a compositional ratio that a product of the formula (1) wherein all $R_2$ are tert-butyl groups, m is 4, n is 0, and one $R_1$ is a methyl group and other three $R_1$ are hydrogen atoms, was 11.7%, one wherein two $R_1$ are methyl groups and other two $R_1$ are hydrogen atoms, was 76.1 %, and one wherein three $R_1$ are methyl groups, and one $R_1$ is a hydrogen atom, was 11.7%.

EXAMPLE 5

[Preparation Example 5]

Into a 1 L four-necked flask equipped with a stirrer, a condenser and a thermometer, 67.92 g of cyclic phenol sulfide corresponding to a cyclic tetramer of the formula (1) wherein all $R_1$ are hydrogen atoms, all $R_2$ are tert-butyl groups, m is 4 and n is 2, 700 ml of DMF and 44.23 g of potassium carbonate, were added and stirred at room temperature for 3.5 hours. Then, 22.71 g of iodomethane was dropwise added, followed by further stirring for 7.5 hours at room temperature. The system was left to stand overnight, and then, insolubles were removed by filtration, followed by concentration under reduced pressure, and the concentrate was added to water. Then, 5 ml of concentrated hydrochloric acid was added, followed by stirring. Precipitated crystals were collected by filtration, and then, dispersion and washing with water were repeated, followed by drying. Obtained crystals were dissolved in 1,600 ml of chloroform, and then 50 ml of trifluoroacetic acid was added, followed by stirring for 4 hours. Washing with water was repeated five times, followed by drying by means of anhydrous sodium sulfate. Concentration under reduced pressure to dryness was carried out to obtain 61.02 g of a mixture of cyclic tetramers of the formula (1) wherein all $R_2$ are tert-butyl groups, m is 4, n is 0, and $R_1$ is a hydrogen atom or a methyl group, as whitish crystals, having the content of an alkali metal such as sodium reduced.
With respect to the obtained whitish crystals, the structure was identified by means of IR. The results of the IR measurement are shown in Fig. 2.
Further, as a result of the HPLC analysis, the crystals were a mixture having such a compositional ratio that a product of the formula (1) wherein all $R_2$ are tert-butyl groups, m is 4, n is 2, and one $R_1$ is a methyl group, and other three $R_1$ are hydrogen atoms, was 8.2%, one wherein two $R_1$ are methyl groups, and other two $R_1$ are hydrogen atoms, was 76.3%, and one wherein three $R_1$ are methyl groups, and one $R_1$ is a hydrogen atom, was 4.0%.

EXAMPLE 6

(Preparation of non-magnetic toner 1)

91 parts of a styrene/acrylate type copolymer resin (tradename: CPR-100, manufactured by Mitsui Chemicals, Inc., acid value: 0.1 mgKOH/g), 1 part of the cyclic phenol sulfide prepared in Example 1, 5 parts of carbon black (tradename: MA-100, manufactured by Mitsubishi Chemical Corporation) and 3 parts of low molecular weight polypropylene (tradename: VISCOL 550P manufactured by Sanyo Chemical Industries, Ltd.) were melted and mixed by means of a heat mixing apparatus (twin screw extrusion kneader) at 130°C. The mixture was cooled and roughly pulverized by a hammer mill and then finely pulverized by a jet mill, followed by classification to obtain a non-magnetic toner having a volume average particle size of $9\pm0.5$ μm.

(Evaluation of non-magnetic toner 1)

This non-magnetic toner and a non-coated ferrite carrier (F-150 manufactured by Powdertech Co., Ltd.) were mixed and shaken in a ratio of toner:carrier of 4 parts by mass: 100 parts by mass thereby to negatively charge the toner. Then, the electric charge amount was measured by a blow off powder electric charge amount measuring apparatus in an atmosphere at a temperature of 25°C and a humidity of 50%. The results are summarized in Table 1.
Further, the time constant ($\tau$) as an index for the charge-rising property was also calculated. For the time constant ($\tau$) in an atmosphere at a temperature of 25°C and a humidity of 50%, the electric charge amount was measured every certain time by a blow off powder electric charge amount measuring apparatus until it reached the saturated electric charge (e.g. Non-Patent Document 1), and $\ln(q^{max}-q)$ was calculated by the following formula, whereupon the relation of the time t and $\ln(q^{max}-q)$ was plotted in a graph, and the time constant $\tau$ was obtained. The results are summarized in Table 1.

$$(q^{max}-q)/(q^{max}-q^{0})=\exp(-t/\tau)$$

In the above formula, $q^{max}$ is the saturated electric charge amount, $q^{0}$ is the initial electric charge amount (in this case, at the time when the charging time was 10 seconds), and t is each measurement time, and the electric charge amount at that time is q.
One having a good charge rising property, will have a time constant being of a smaller value. The unit of the time constant is seconds.
In the same manner, the electric charge amount and the time constant were evaluated also with respect to a

case where the non-magnetic toner was mixed with a silicon-coated type ferrite carrier (F96-150 manufactured by Powdertech Co., Ltd.). The results are summarized in Table 1.

EXAMPLE 7

(Preparation and evaluation of non-magnetic toner 2)

A non-magnetic toner 2 was prepared in the same manner as in Example 6 except that in Example 6, the cyclic phenol sulfide prepared in Example 1 was changed to the cyclic phenol sulfide prepared in Example 2, and the electric charge amount and the time constant in an atmosphere at a temperature of 25°C and a humidity of 50% were evaluated. The results are summarized in Table 1.

COMPARATIVE EXAMPLE 1

(Preparation and evaluation of comparative non-magnetic toner)

For the purpose of comparison, a comparative non-magnetic toner was prepared in the same manner as in Example 6 except that in Example 6, the cyclic phenol sulfide prepared in Example 1 was changed to a salt of 3,5-tert-butyl salicylic acid and zinc (comparative compound 1), and the electric charge amount and the time constant in an atmosphere at a temperature of 25°C and a humidity of 50%, were evaluated by a blow off powder electric charge amount measuring apparatus. The results are summarized in Table 1.

[Table 1]

| Toner | Carrier F-150 | | Carrier F96-150 | |
|---|---|---|---|---|
| | Electric charge amount (μc/g) | Time constant τ (s) | Electric charge amount (μc/g) | Time constant τ (s) |
| Ex. 6 | -27.6 | 119 | -24.3 | 105 |
| Ex. 7 | -27.1 | 122 | -23.9 | 106 |
| Comp. Ex. 1 | -23.0 | 200 | -15.0 | 108 |

As is evident from Table 1, it has been found that by a toner using a charge controlling agent comprising, as an effective component, cyclic phenol sulfide represented by the formula (1) of the present invention, the rising property of electrostatic charge is improved, and the electric charge amount becomes high.

EXAMPLE 8

(Preparation and Evaluation of non-magnetic toner 3)

A non-magnetic toner 3 was prepared in the same manner as in Example 6 except that in Example 6, the cyclic phenol sulfide prepared in Example 1 was changed to the cyclic phenol sulfide prepared in Example 3. The electric charge amount and the time constant in an atmosphere at a temperature of 25°C and a humidity of 50%, were evaluated by a blow off powder electric charge amount measuring apparatus, and as a result, results equivalent to the results in Examples 6 and 7 were obtained.

Further, the environmental stability at a high temperature and a high humidity (30°C, 85% RH) was evaluated. The environmental stability was evaluated by a decreasing rate of the saturated electric charge amount at a high temperature and high humidity against the saturated electric charge amount in an atmosphere at a temperature of 25°C and a humidity of 50%. As a result of the measurement, the decreasing rate of a saturated electric charge amount was at most 5%.

EXAMPLE 9

(Preparation and evaluation of non-magnetic toner 4)

A non-magnetic toner 4 was prepared in the same manner as in Example 6 except that in Example 6, the cyclic phenol sulfide prepared in Example 1 was changed to the cyclic phenol sulfide prepared in Example 4.

The electrical charge amount and the time constant in an environment at a temperature of 25°C and a humidity of 50% were evaluated by a blow off powder electric charge amount measuring apparatus, whereby results equivalent to the results in Examples 6 and 7 were obtained.

Further, the environmental stability at a high temperature and a high humidity (30°C, 85% RH) was evaluated. The environmental stability was evaluated by a decreasing rate of the saturated electrical charge amount at a high temperature and a high humidity against the saturated electrical charge amount in an environment at a temperature of 25°C and a humidity of 50%. As a result of the measurement, the decreasing rate of the saturated electrical charge amount was at most 5%.

EXAMPLE 10

(Preparation and evaluation on non-magnetic toner 5)

A non-magnetic toner 5 was prepared in the same manner as in Example 6 except that in Example 6, the cyclic phenol sulfide prepared in Example 1 was changed to the cyclic phenol sulfide prepared in Example 5.

The electric charge amount and the time constant in an environment at a temperature of 25°C and a humidity of 50% were evaluated by a blow off powder electric charge amount measuring apparatus, and as a result, results equivalent to the results in Examples 6 and 7, were obtained.

Further, the environmental stability at a high temperature and a high humidity (30°C, 85% RH) was evaluated. The environmental stability was evaluated by a decreasing rate of the saturated electric charge amount at a high temperature and a high humidity against the saturated electric charge amount in an atmosphere at a temperature of 25°C and a humidity of 50%. As a result of the measurement, the decreasing rate of the saturated electric charge amount was at most 5%.

As is evident from the above results, by carrying out an operation to reduce the content of an alkali metal such as sodium, it is possible to impart a high charging ability and high environmental stability to a toner wherein such a compound is used as a charge controlling agent.

EXAMPLE 11

[Preparation of resin dispersion]

80 parts of a polyester resin (DIACRON ER-561 manufactured by Mitsubishi Rayon Co., Ltd.), 320 parts of ethyl acetate and 32 parts of isopropyl alcohol were mixed, and while stirring at from 5,000 to 10,000 rpm by means of a homogenizer (foamless mixer NGM-0.5TB manufactured by Beryu Co., Ltd.), a 0.1 mass% aqueous ammonia was dropwise added in a proper amount for phase-change emulsification, and removal of the solvent was carried out under reduced pressure by an evaporator to obtain a resin dispersion. The volume average particle size of resin particles in this dispersion was 0.2 $\mu$m (the concentration of resin particles was adjusted to be 20 mass% with ion-exchanged water).

[Preparation of charge controlling agent dispersion]

0.2 part of sodium dodecyl benzene sulfonate, 0.2 part of Sorbon T-20 (manufactured by Toho Chemical Industry Co., Ltd.) and 17.6 parts of ion-exchanged water were mixed and dissolved, and further, 2.0 parts of the cyclic phenol sulfide prepared in Example 1 and zirconia beads (particle size of beads: 0.65 mm in diameter, corresponding to 15 ml) were added, followed by dispersion for 3 hours by a paint conditioner (Red Devil No. 5400-5L manufactured by UNION N.J. (USA)). By using a sieve, zirconia beads were removed, followed by adjustment with ion-exchanged water to obtain a 10 mass% charge controlling agent dispersion.

[Preparation of polymerized toner]

Into a reactor equipped with a thermometer, a pH meter and a stirrer, 125 parts of the above resin dispersion, 1.0 part of a 20 mass% sodium dodecylbenzene sulfonate aqueous solution and 125 parts of ion-exchanged water were added and stirred for 30 minutes at a rotational speed of 150 rpm while controlling the liquid temperature to be 30°C. A 1 mass% nitric acid aqueous solution was added to adjust the pH to be 3.0, followed by further stirring for 5 minutes. While dispersing the mixture by a homogenizer (ULTRA TURRUX T-25, manufactured by IKA Japan), 0.125 part of polyaluminum chloride was added, and after raising the liquid temperature to 50°C, dispersion was carried out for further 30 minutes. 62.5 parts of the above resin dispersion and 4.0 parts of the above charge controlling agent dispersion were added, and then a 1 mass% nitric acid aqueous solution was added to adjust the pH to be 3.0, followed by dispersion for further 30 minutes. While stirring at from 400 to 700 rpm by using a stirrer, 8.0 parts of a 5 mass% sodium hydroxide

aqueous solution was added, and stirring was continued until the volume average particle size of the toner became 9.5 μm. After raising the liquid temperature to 75°C, stirring was continued for further two hours, and after confirming that the volume average particle size became 6.0 μm, and the particle shape became spherical, quenching was carried out by using ice water. The particles were collected by filtration and subjected to dispersion and washing with ion-exchanged water. The dispersion and washing were repeated until the electrical conductivity of the filtrate after dispersion became at most 20 μS/cm. Then, drying was carried out by a dryer at 40°C to obtain toner particles.

The obtained toner was sieved by a sieve with 166 mesh (aperture: 90 μm) to obtain a toner for evaluation.

[Evaluation]

Two parts of the obtained toner for evaluation and 100 parts of a silicon-coated ferrite carrier (F96-150 manufactured by Powdertech Co., Ltd.) were mixed and shaken to negatively charge the toner, and then measurement of a saturated electric charge amount was carried out in an atmosphere at a temperature of 25°C and a humidity of 50% by a blow off powder electric charge amount-measuring apparatus. As a result, the saturated electric charge amount was -37.7 μC/g.

COMPARATIVE EXAMPLE 2

For the purpose of comparison, a toner was prepared and its saturated electric charge amount was measured under the same conditions as in Example 11 except that in Example 11, the operation of adding the charge controlling agent dispersion was omitted. As a result, the saturated electric charge amount was -20.5 μC/g.

COMPARATIVE EXAMPLE 3

For the purpose of comparison, a toner was prepared and its saturated electric charge amount was measured under the same conditions as in Example 11 except that in Example 11, the cyclic phenol sulfide prepared in Example 1 was changed to the cyclic tetramer (comparative compound 2) of the formula (1) wherein all $R_2$ are tert-butyl groups, m is 4, n is 0, and all $R_1$ are hydrogen atoms. As a result, the saturated electric charge amount was -21.2 μC/g.

As is evident from the above results, it has been found that a polymerized toner comprising, as an effective component, cyclic phenol sulfide represented by the formula (1) of the present invention exhibits an excellent charging performance.

That is, it is possible to impart a high charging performance to a polymerized toner by using a charge controlling agent comprising, as an effective component, cyclic phenol sulfide represented by the formula (1) of the present invention.

INDUSTRIAL APPLICABILITY

The cyclic phenol sulfide represented by the formula (1) of the present invention has an excellent charging performance, and a charge controlling agent containing such a compound as an effective component has a charging performance which is distinctly higher than conventional charge controlling agents. Further, it is possible to impart a high environmental stability by reducing the content of an alkali metal such as sodium. Further, it is most suitable for a color toner, particularly for a polymerized toner. Furthermore, it is possible to provide a very useful toner which does not contain a heavy metal such as a chromium compound which brings about an environmental problem.

The entire disclosure of Japanese Patent Application No. 2010-206201 filed on September 15, 2010 including specification, claims, drawings and summary is incorporated herein by reference in its entirety.

## Claims

1. A charge controlling agent comprising, as an effective component, at least one type of cyclic phenol sulfide represented by the following formula (1):

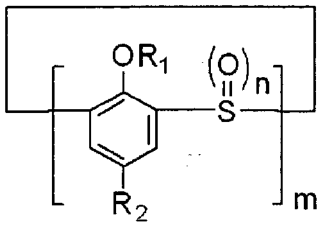

(1)

wherein $R_1$ is a hydrogen atom, a $C_{1-8}$ linear or branched alkyl group which may have a substituent, a substituted or unsubstituted aromatic hydrocarbon group, or a substituted or unsubstituted condensed polycyclic aromatic group, $R_2$ is a $C_{1-8}$ linear or branched alkyl group which may have a substituent, a substituted or unsubstituted aromatic hydrocarbon group, a substituted or unsubstituted aromatic heterocyclic group, or a substituted or unsubstituted condensed polycyclic aromatic group, m is an integer of from 4 to 9, and n is an integer of 0, 1 or 2, wherein the plurality of $R_1$ present in one molecule, may be the same or different from one another, provided that at least one of them is a hydrogen atom but not all of them are hydrogen atoms.

2. The charge controlling agent according to Claim 1, wherein in the formula (1), n is 0.

3. The charge controlling agent according to Claim 1 or 2, wherein in the formula (1), m is 4.

4. The charge controlling agent according to Claim 2 or 3, which comprises, as an effective component, a mixture of three types of cyclic phenol sulfide of the formula (1), wherein m is 4, and n is 0.

5. The charge controlling agent according to any one of Claims 1 to 4, wherein in the formula (1), $R_1$ is a hydrogen atom, or a $C_{1-4}$ linear or branched alkyl group which has no substituent.

6. The charge controlling agent according to any one of Claims 1 to 5, wherein in the formula (1), $R_2$ is a $C_{1-4}$ linear or branched alkyl group which may have a substituent.

7. A toner comprising the charge controlling agent as defined in any one of Claims 1 to 6, and a colorant and a binder resin.

8. A polymerized toner comprising the charge controlling agent as defined in any one of Claims 1 to 6, and a colorant and a binder resin.

Fig. 1

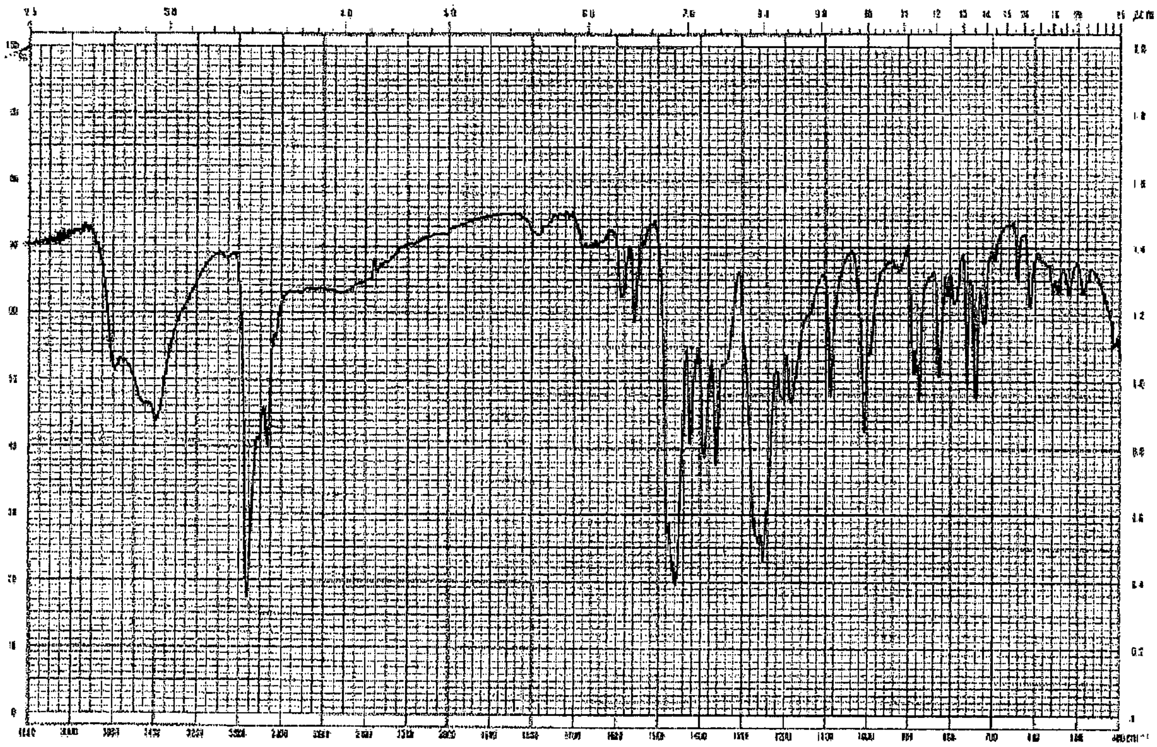

Fig. 2

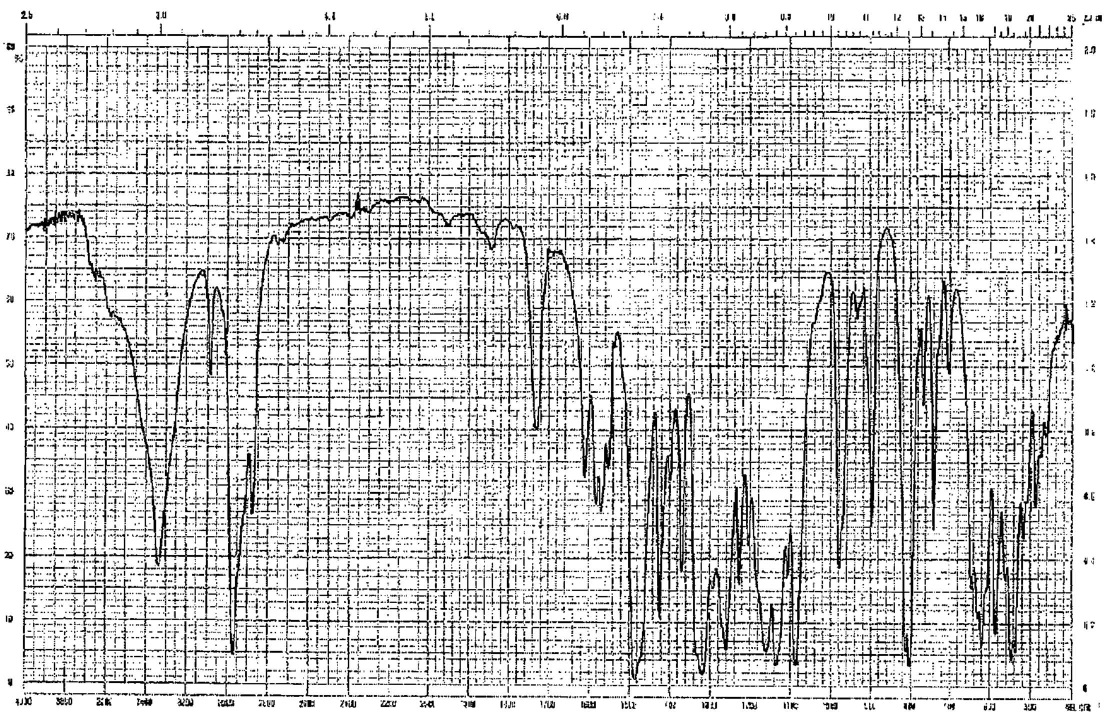

## INTERNATIONAL SEARCH REPORT

International application No.
PCT/JP2011/070901

A. CLASSIFICATION OF SUBJECT MATTER
*G03G9/097*(2006.01)i, *C07C321/28*(2006.01)i, *G03G9/087*(2006.01)i

According to International Patent Classification (IPC) or to both national classification and IPC

B. FIELDS SEARCHED

Minimum documentation searched (classification system followed by classification symbols)
G03G9/097, C07C321/28, G03G9/087

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CAplus(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 10-168078 A (Cosmo Research Institute),<br>23 June 1998 (23.06.1998),<br>claims; paragraphs [0003], [0029]<br>(Family: none) | 1-6<br>7,8 |
| X<br>Y | JP 2010-113117 A (Konica Minolta Business Technologies, Inc.),<br>20 May 2010 (20.05.2010),<br>claim 4; paragraph [0030], A5<br>(Family: none) | 1-6<br>7,8 |
| Y | JP 2003-295522 A (Toda Kogyo Corp.),<br>15 October 2003 (15.10.2003),<br>claims<br>(Family: none) | 7 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>04 November, 2011 (04.11.11) | Date of mailing of the international search report<br>15 November, 2011 (15.11.11) |
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

International application No.
PCT/JP2011/070901

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2010-117631 A (Konica Minolta Business Technologies, Inc.), 27 May 2010 (27.05.2010), claims; examples (Family: none) | 7,8 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 61069073 A
- JP 57111541 A
- JP 62094856 A
- US P4767688 A
- JP 61003149 A
- JP 3313871 B
- JP 2003295522 A
- WO 2007111346 A
- WO 2007119797 A
- JP 58009154 A
- JP 10081680 A
- WO 9809959 A
- JP 2010206201 A


### Non-patent literature cited in the description


- *DENSHI SHASHIN GAKKAISHI,* 1988, vol. 27 (3), 307